# EUROPEAN PATENT SPECIFICATION

(11) **EP 3 375 880 B1**
(45) Date of publication and mention of the grant of the patent: **25.12.2019**
(21) Application number: 16860246.4
(22) Date of filing: 27.10.2016
(51) Int. Cl.: C12P 13/04, C12N 15/70, C12N 15/81, C12P 7/64, C12P 13/00, C12N 9/02, C12N 9/10

(54) **METHOD FOR PRODUCING HEAVY CHAIN AMINOCARBOXYLIC ACID**
VERFAHREN ZUR HERSTELLUNG VON SCHWERKETTIGER AMINOCARBONSÄURE
PROCÉDÉ DE PRODUCTION D'UN ACIDE AMINOCARBOXYLIQUE À CHAÎNE LOURDE

(30) Priority: 27.10.2015 KR 20150149254
(43) Date of publication of application: 19.09.2018
(73) Proprietor: Korea Research Institute of Bioscience and Biotechnology, Daejeon 34141 (KR)
(72) Inventor: AHN, Jung Oh, Daejeon 34141 (KR); LEE, Hong Weon, Daejeon 34141 (KR); PARK, Gyu Yeon, Daejeon 34141 (KR); JANG, Min Jeong, Daejeon 34141 (KR); JEON, Woo Young, Daejeon 34141 (KR)
(74) Representative: Cabinet Laurent & Charras
(86) International application number: PCT/KR2016/012172
(87) International publication number: WO 2017/074063

(56) References cited:
- WO-A1-2014/201474
- DE-A1-102007 060 705
- JP-A- 2006 271 378
- JP-A- 2011 055 790
- KR-B1- 101 145 405
- US-B2- 8 530 206
- US-B2- 9 012 227
- JI-WON SONG ET AL: "Microbial Synthesis of Medium-Chain [alpha],[omega]-Dicarboxylic Acids and [omega]-Aminocarboxylic Acids from Renewable Long-Chain Fatty Acids", ADVANCED SYNTHESIS & CATALYSIS, vol. 356, no. 8, 26 May 2014 (2014-05-26), pages 1782-1788, XP055248703, DE ISSN: 1615-4150, DOI: 10.1002/adsc.201300784
- RIZZO: 'Fatty Aldehyde and Fatty Alcohol Metabolism: Review and Importance for Epidermal Structure and Function' BIOCHIMICA ET BIOPHYSICA ACTA vol. 1841, 2014, pages 377 - 389, XP055379649

<110> Korea Research Institute of Bioscience and Biotechnology
<120> METHOD FOR PRODUCTION OF MEDIUM CHAIN AMINOCARBOXYLIC ACID
<130> 2016-OPA-8750PCT
<150> KR 10-2015-0149254
   <151> 2015-10-27
<160> 106
<170> KopatentIn 2.0
<210> 1
   <211> 1602
   <212> DNA
   <213> Yarrowia lipolytica
<220>
   <221> gene
   <222> (1)..(1602)
   <223> FALDH1 gene
<400> 1
   atgtcctggg aaacaatcac tcctcctacg ccaatcgata cgtttgacag caacttgcaa      60
   cgtcttcgag actctttcga gaccggcaag ctcgactctg tcgactaccg tctcgagcag     120
   ctgcgaaccc tgtggttcaa gttctacgac aacctcgaca acatctacga ggcggtcacc     180
   aaggatctcc atcgacccag gttcgaaacc gagctcaccg aggtactgtt tgttcgagac     240
   gagttctcca ccgtcatcaa gaacctgcga aagtgggtca aggaagaaaa ggtggagaac     300
   cccggaggcc ccttccagtt tgccaacccc cgaatccgac ccgttcctct gggagtggtg     360
   ctggtcatca ctccctggaa ctaccccgtc atgctcaaca tctcacctgt gattgccgcc     420
   attgctgccg gctgtcccat cgtgctcaag atgtccgagc tgtctcccca cacttccgct     480
   gttcttggcc gaatcttcaa ggaggccctg gaccccggta tcatccaggt tgtttacgga     540
   ggtgtccccg agaccaccgc ccttcttacc cagcattggg acaagatcat gtacaccgga     600
   aacggagccg ttggtcgaat catcgcccag gccgcggtca agaacctgac tcctctagct     660
   cttgagcttg gtggcaagtc acccgtgttc atcacttcca actgcaagag cgttatgacg     720
   gccgctcggc gaatcgtgtg gggcaagttt gtcaacgccg gccagatctg tgtcgctcca     780
   gactacattc tggttgctcc cgaaaaggag gccgagctcg tcgcttgtat caaggaggtg     840
   ctccaagaac gatacggctc caagagagac gcccaccacc ccgatctgtc ccatatcatt     900
   tccaagcccc attggaagcg tattcacaac atgatcgccc agaccaaggg agacatccag     960
   gtgggtggac tcgagaacgc cgacgaagac caaaagttca tccagcccac aatcgtctcc    1020
   aacgttccag atgacgacat tctcatgcag gacgagattt tcggacccat catccccatc    1080
   atcaagcccc gaaccctcgg ccagcaggtt gattacgtca caagaaacca tgacaccccc    1140
   ctggccatgt acatcttctc tgacgacccc aaggaggtgg actggctaca gacccgaatc    1200
   cgagctggtt ctgtaaacat caacgaggtc attgagcagg tcggactggc ctctctgcct    1260
   ctcagtggag ttggagcttc cggaaccgga gcataccatg gaaaattctc cttcgatgtc    1320
   ttcacccaca agcaggccgt tatgggacag cccacctggc ccttctttga atacctcatg    1380
   tattaccggt accctcctta ctccgagtac aagatgaagg tgctccgaac cctgttccca    1440
   ccggttctga ttcctcgaac cggccgaccc gacgctactg ttcttcagcg agttctcggc    1500
   aacaagctgc tttggatcat tattgccgcc cttgttgcgt acgccaaacg aaatgagctg    1560
   ctcatcacca ttgctcagat tatgtcggtg tttattaagt ag                        1602
<210> 2
   <211> 1566
   <212> DNA
   <213> Yarrowia lipolytica
<220>
   <221> gene
   <222> (1)..(1566)
   <223> FALDH2 gene
<400> 2
   atgtcagagt tcgattggga gtcaattttg ccggcaacac cactaggtga gatcgagaag      60
   gatattcaaa ccctacgaca gggcttcagg tccggaaaga cgctggattt gaacttcagg     120
   cttgaccaga ttcgtaagct tttctatgct ctctatgata atgtcgatgc gatcaaagaa     180
   gcaattcata aggatctcgg acgtccggtc ttcgagactg aactttgcga gatctccttt     240
   cagtggggtg aattcaataa tgtcgtttct aacttgaaga aatgggcagc tgatgagacg     300
   gtgaagggaa ccaccattca atacactctc acccggccaa agattagaaa gcgtccactt     360
   ggtaccgtcc ttatcatatc tccttggaac tacccatttg ttctgaccat ctctcccctg     420
   cttgctgctc tagcggcagg aaatacggtg gccctaaagt tctccgaaat gtgcccacat     480
   acatcgcttt tgctgggaaa gttgtgcaca gaggcacttg ataaagaaat tttcaaggca     540
   tttcagggag gcgttccggt agtgtcggag attctcaagt acaagttcga caaaatcatg     600
   tacactggaa atcatcgagt tggcaagatc atcttggacg cagctaacaa atacctcacc     660
   cccgttattt tggagcttgg aggcaaatca ccagtcttcg tgactaagaa ttgccaaaac     720
   gtatctcttg ctgccaagcg tgctctgtgg ggtaaactgg tcaacgctgg acaaacatgc     780
   gttgcccccg attacatcat cgtcgagcct gaggtcgaac aggagtttat caaagcttgc     840
   cagtactggg ttgagaagtt ctaccgaggt ggagttgact ctgatcataa ggacttcact     900
   catattgcaa cacctggaca ttggagacga ttgacatcca tgcttgccca gacagaggga     960
   aatatcatca caggcggaaa ttcggacgag aaatcacggt ttcttgctcc cacagttgtt    1020
   gcgaaagttc ctgatggtga ttctttgatg aatgatgaga tctttggccc tatcctgccc    1080
   atcctgacag ccagatccgt tgacgaaggt attcgctatg ttcatgagaa tcacgacact    1140
   cccctggcca tgtatgtctt tactgataat gcatcagaag gagagtatat ccaatctcaa    1200
   atcaactcag gtggcctgat attcaatgat agtcttgttc acgttggctg cgtgcaggcg    1260
   ccttttggtg gtgtcggcca atccggctat gggtcttatc acggcgaaga ttccttcttg    1320
   gctttttcac acaggcagac tttcatgaag cagccccatt tcatcgaacg accaatggcg    1380
   atcagatatg ccccctacac tagtcgaaaa caaaaggctg tccagggtag tctagctgct    1440
   ccatcttttc ctcgaacagg aaaggttgac cgctccctgt tggagcggat atttggtaag    1500
   ctatggttct gggtgatcgt tttagggcta ggagcagcca gtttgaagtc aggaattttc    1560
   ttatga                                                               1566
<210> 3
   <211> 1590
   <212> DNA
   <213> Yarrowia lipolytica
<220>
   <221> gene
   <222> (1)..(1590)
   <223> FALDH3 gene
<400> 3
   atgactacca ctgccacaga gacccccacg acaaacgtga cccccaccac gtcactgccc      60
   aaggagaccg cctccccagg agggaccgct tctgtcaaca cgtcattcga ctgggagagc     120
   atctgcggca agacgccgtt ggaggagatc gagtcggaca tttcgcgtct caaaaagacc     180
   ttccgatcgg gcaaaactct ggatctggac taccgactcg accagatccg aaacctggcg     240
   tatgcgatcc gcgataacga aaacaagatc cgcgacgcca tcaaggcgga cctgaaacga     300
   cctgacttcg aaaccatggc ggccgagttc tcggtccaga tgggcgaatt caactacgtg     360
   gtcaaaaacc tgccgaaatg ggtcaaggac gaaaaagtca agggaaccag catggcgtac     420
   tggaactcgt cgccaaagat ccggaaacgg cccctgggct ccgtgcttgt catcacgccc     480
   tggaactacc cactgattct ggccgtgtcg cctgttctgg gcgccattgc cgcaggcaac     540
   accgtggcgc tgaaaatgtc agaaatgtca cccaacgcgt caaaggtgat tggcgacatt     600
   atgacagctg ccctggaccc ccagctcttt caatgcttct tcggaggagt ccccgaaacc     660
   accgagatcc tcaaacacag atgggacaag atcatgtaca ccggaaacgg caaagtgggc     720
   cgaatcatct gtgaggctgc caacaagtac ttgacacctg tggagctcga actcggagga     780
   aagtcgcctg ttttcgtcac caaacactgc tccaacctgg aaatggccgc ccgccgaatc     840
   atctggggca aattcgtcaa cggaggacaa acctgcgtgg ctccagacta cgttctggtg     900
   tgtcccgagg tccacgacaa atttgtggct gcctgtcaaa aggtgctgga caagttctac 960
   cctaacaact ctgccgagtc cgagatggcc catatcgcca cccctctcca ttacgagcgt 1020
   ttgacgggcc tgctcaattc cacccgaggt aaggtcgttg ctggaggcac tttcaactcg 1080
   gccacccggt tcattgctcc tacgattgtc gacggagtgg atgccaacga ttctctgatg 1140
   cagggagaac tgtttggtcc tcttctcccc attgtcaagg ccatgagcac cgaggctgcc 1200
   tgcaactttg tgcttgagca ccaccccacc cccctggcag agtacatctt ttcagataac 1260
   aattctgaga ttgattacat ccgagatcga gtgtcgtctg gaggtctcgt gatcaacgac 1320
   actctgatcc acgtgggatg cgtacaggcg ccctttggag gtgtcggaga cagtggaaat 1380
   ggaggatacc atggcaagca cactttcgat ttgttcagcc attctcagac ggtcctcaga 1440
   caacccggat gggtcgaaat gctgcagaag aaacggtatc ctccgtacaa caagagcaac 1500
   gagaagtttg tccggagaat ggtggtcccc agccctggtt ttccccggga gggtgacgtg 1560
   agaggatttt ggtcgagact cttcaactag 1590
<210> 4
   <211> 1560
   <212> DNA
   <213> Yarrowia lipolytica
<220>
   <221> gene
   <222> (1)..(1560)
   <223> FALDH4 gene
<400> 4
<210> 5
   <211> 2034
   <212> **DNA**
   <213> Yarrowia lipolytica
<220>
   <221> gene
   <222> (1)..(2034)
   <223> ACO1 gene
<400> 5
   atggccaagg agcgaggtaa gactcaattc actgtccgag atgtgaccaa cttcctcaat      60
   ggtggagaag aagagaccca gattgtcgag aagatcatga gcagtattga acgtgatcca     120
   gtactgtctg tcactgctga ctacgactgc aaccttcagc aggcccgaaa acagaccatg     180
   gagcgggtgg ctgctctgtc gccttatctg gtcaccgata ctgagaagct atctctgtgg     240
   cgtgcgcaac tgcatggaat ggttgatatg tctactcgta cgcggttgtc gatccacaac     300
   aacctgttca ttggttccat caggggatct ggtactcctg aacagttcaa gtactgggtc     360
   aagaagggag cggtggctgt taagcagttc tatggatgct ttgccatgac agagttgggc     420
   catggaagca acctcaaggg actagagaca accgccactt atgaccagga cagtgaccag     480
   ttcattatca acactcctca tattggtgct accaagtggt ggattggcgg tgcagcccac     540
   acttccaccc attgtgtttg tttcgcgaaa ctgattgtgc atggcaagga ctatggtact     600
   cgaaactttg tggtacctct ccgaaatgtc cacgatcaca gtctcaaggt cggtgtttca     660
   attggagaca ttggaaagaa gatgggcaga gatggtgttg acaatggctg gatccagttc 720
   accaatgttc gaatccccag acagaacatg ctaatgagat atgccaaggt gtctgatact 780
   ggagtggtaa ccaaacccgc tcttgaccaa ctcacttatg gagccctcat tcgaggtcga 840
   gtgtccatga ttgccgactc gttccacgtc tccaaacgat tcctcacaat tgctcttcgg 900
   tacgcttgtg tccgacgaca gtttggaacc tctggagaca ctaaggagac caagatcatc 960
   gactaccctt accaccagcg acgattgctg cctcttctgg cctactgcta cgctatgaag 1020
   atgggtgctg atgaggctca gaagacttgg attgagacca ccgatcgaat tctggctctc 1080
   aatcccaacg accccgccca gaagaacgat ctggagaagg ccgtcaccga cacaaaggag 1140
   ctgtttgctg cgtctgcagg aatgaaggca tttaccacgt ggggatgtgc caaaatcatt 1200
   gatgagtgcc gacaggcctg tggaggtcat ggatactctg gatataacgg atttggccag 1260
   ggctacgctg actgggttgt ccagtgtacc tgggaaggag acaacaacgt tctgtgtctg 1320
   tcaatgggcc gagggctggt tcagtcagct ctacagattt tggctggaaa gcacgtcggt 1380
   gcttctattc agtacgtagg agacaagtct aaaatctccc agaacggcca gggtaccccc 1440
   agagagcaac ttctgtcccc cgagtttcta gtagaagctt tcagaacggc ttctcgaaac 1500
   aacattctca gaaccaccga taaataccaa gagcttgtca aaactctcaa tcccgaccag 1560
   gcctttgagg agctgtctca gcagagattc cagtgtgctc gaatccacac acgacagcat 1620
   cttatctctt cattctatgc ccgaattgcc actgccaaag acgatatcaa gccccatctg 1680
   ctgaaactgg ccaatctgtt tgccctctgg tcaattgagg aggacactgg aatcttcctg 1740
   cgggagaaca tcctcacccc tggagacatt gacctgatca acagtcttgt ggacgagctc 1800
   tgtgttgcag ttcgagatca ggtaattgga ctcactgatg cctttggtct ctctgacttc 1860
   ttcattaacg ctcccatcgg ctcctacgat ggtaatgttt acgaaaagta ctttgccaag 1920
   gtcaaccagc aaaaccccgc tactaaccct cgtcctccct actacgagtc gactctcaag 1980
   cccttcttgt tccgagaaga ggaggacgat gaaatttgcg atctcgatga gtga 2034
<210> 6
   <211> 2103
   <212> DNA
   <213> Yarrowia lipolytica
<220>
   <221> gene
   <222> (1)..(2103)
   <223> ACO2 gene
<400> 6
   atgaacccca acaacactgg caccattgaa atcaacggta aggagtacaa caccttcacc 60
   gagccccccg tggccatggc tcaggagcga gccaagacct ccttccccgt gcgagagatg 120
   acctacttcc tcgacggtgg cgagaagaac accctcaaaa acgagcagat catggaggag      180
   attgagcgag accctctttt caacaacgac aactactacg atctcaacaa ggagcagatc      240
   cgagagctca ccatggagcg agtcgccaag ctgtctctgt ttgtgcgtga tcagcccgag      300
   gacgacatca agaagcgatt tgctctcatt ggtatcgccg atatgggaac ctacacccga      360
   cttggtgtcc actacggcct cttctttggc gccgtccgag gtaccggaac tgccgagcag      420
   tttggccact ggatctccaa gggagccgga gacctgcgaa agttctacgg atgtttctcc      480
   atgaccgagc tgggccatgg ctccaacctg gctggtctcg agaccaccgc catctacgat      540
   gaggagaccg acgagttcat catcaacacc cctcacattg ccgccaccaa gtggtggatt      600
   ggaggagccg cccacaccgc cacccacact gtcgtgttcg cccgactcat tgtcaagggc      660
   aaggactacg gtgtcaagac ctttgttgtc cagctgcgaa acatcaacga ccacagcctc      720
   aaggtcggta tctctattgg tgatatcgga aagaagatgg gccgagacgg tatcgataac      780
   ggatggatcc agttcaccaa cgtgcgaatc ccccgacaga acctgctcat gaagtacaca      840
   aaggtcgacc gagagggtaa cgtgacccag cctcctctgg ctcagcttac ctacggttct      900
   cttatcactg gtcgagtctc catggcctct gattctcacc aggtcggaaa gcgattcatc      960
   accattgctc tgcgatacgc ctgcattcga cgacagttct ccaccacccc cggccagccc     1020
   gagaccaaga tcatcgacta cccctaccat cagcgacgac ttctgcctct tctggcctat     1080
   gtctatgctc ttaagatgac tgccgatgag gttggagctc tcttctcccg aaccatgctt     1140
   aagatggacg acctcaagcc cgacgacaag gccggcctca atgaggttgt ttccgacgtc     1200
   aaggagctct tctccgtctc cgccggtctc aaggccttct ccacctgggc ttgtgccgac     1260
   gtcattgaca agacccgaca ggcttgcggt ggccacggtt actctggata caacggtttc     1320
   ggccaggcct acgccgactg ggttgtccag tgcacctggg agggtgacaa caacattctc     1380
   accctttctg ccggccgagc tcttatccag tctgccgttg ctctgcgaaa gggcgagcct     1440
   gttggtaacg ccgtttctta cctgaagcga tacaaggatc tggccaacgc taagctcaat     1500
   ggccgatctc tcaccgaccc caaggtcctc gtcgaggcct gggaggttgc tgccggtaac     1560
   atcatcaacc gagccaccga ccagtacgag aagctcattg gcgagggtct taacgccgac     1620
   caggcctttg aggttctgtc tcagcagcga ttccaggccg ccaaggtcca cacacgacga     1680
   cacctcattg ccgctttctt ctcccgaatt gacaccgagg ctggcgaggc catcaagcag     1740
   cccctgctta acctggctct gctgtttgcc ctgtggtcca tcgaagagga ctctggtctg     1800
   ttcctgcgag agggcttcct cgagcccaag gatatcgaca ccgtcaccga gctcgtcaac     1860
   aagtactgca ccactgtgcg agaggaggtc attggctaca ccgatgcctt caacctgtcc     1920
   gactacttca tcaacgctcc tattggatgc tacgatggtg acgcttaccg acactacttc     1980
   cagaaggtca acgagcagaa ccctgcccga gacccccgac ctccttacta cgcctctact     2040
   ctcaagccct tccttttccg agaggaggag gatgatgaca tttgcgagct tgatgaggaa 2100
   tag 2103
<210> 7
   <211> 2103
   <212> DNA
   <213> Yarrowia lipolytica
<220>
   <221> gene
   <222> (1)..(2103)
   <223> ACO3 gene
<400> 7
   atgatctccc ccaacctcac agctaacgtc gagattgacg gcaagcagta caacaccttc      60
   acagagccac ccaaggcgct cgccggcgag cgagccaagg tcaagttccc catcaaggac     120
   atgacggagt ttctgcacgg tggcgaggag aacgtgacca tgatcgagcg actgatgacg     180
   gagctcgagc gagaccccgt gctcaacgtg tcgggcgact acgacatgcc caaggagcag     240
   ctgcgagaga cggccgtggc gcgaattgcg gcgctgtccg gccactggaa gaaggacaca     300
   gaaaaggagg cgctgctgcg gtcccagctg cacggcattg tggacatggg cacccgaatc     360
   cgactcggtg tgcacacggg cctgttcatg ggcgccatcc ggggttccgg caccaaggag     420
   cagtacgact actgggtgcg aaagggcgcc gcggacgtca agggcttcta cggctgcttt     480
   gctatgaccg agctgggcca tggctccaac gtggccggtc ttgagaccac cgccacctac     540
   atccaggaca cggacgagtt catcatcaac acccccaaca ctggagccac caagtggtgg     600
   attggaggag ccgcccactc ggccacccac accgcctgct ttgctcgtct gcttgtcgac     660
   ggcaaggact acggcgtcaa gatctttgtt gtccagctgc gagacgtctc ttctcactct     720
   ctcatgcccg gcatcgctct cggcgacatt ggaaagaaga tgggccgaga cgccatcgac     780
   aacggctgga tccagttcac caatgtgcga atcccccgac agaacatgct catgaagtac     840
   gccaaggtct cgtctaccgg caaggtgtcg cagcctcctc tggcccagct cacctacggc     900
   gctctcattg gcggccgagt caccatgatt gccgactcct tctttgtctc ccagcgattc     960
   atcaccattg ctctgcgata cgcctgtgtg cgacgacagt ttggcaccac ccccggccag    1020
   cccgagacta agatcatcga ctacccctac catcagcgac gtctgctgcc tcttctggcc    1080
   ttcacctacg ccatgaagat ggccgccgac cagtcccaga ttcagtacga tcagaccacc    1140
   gatctgctgc agaccatcga ccctaaggac aagggcgctc tgggcaaggc cattgtcgac    1200
   ctcaaggagc tgtttgcctc ttctgctggt ctcaaggcct tcaccacctg gacctgtgcc    1260
   aacatcattg accagtgccg acaggcctgc ggtggccacg gctactctgg ctacaacggc    1320
   tttggccagg cctacgccga ctgggttgtc cagtgcacct gggagggtga caacaacgtc    1380
   ctgtgtctgt ccatgggccg aggtctcatc cagtcgtgtc tgggccaccg aaagggtaag 1440
   cctctgggct cttctgtcgg ctacctggct aacaagggtc ttgagcaggc tactctgagc 1500
   ggccgagacc tcaaggaccc caaggttctc atcgaggcct gggagaaggt cgccaacggc 1560
   gccatccagc gggccactga caaatttgtc gagctcacca agggcggcct ctctcctgac 1620
   caggcctttg aggagctgtc gcagcagcga ttccagtgtg ccaagatcca cacccgaaag 1680
   cacctggtga ctgccttcta cgagcgaatc aacgcctctg cgaaggccga cgtcaagcct 1740
   tacctcatca acctcgccaa cctcttcact ctgtggtcca ttgaggagga ctctggtctc 1800
   ttcctgcgag agggtttcct gcagcccaag gacattgacc aggtgactga gctggtgaac 1860
   cactactgca aggaggttcg agaccaggtt gccggctaca ccgatgcctt tggtctgtct 1920
   gactggttca tcaacgctcc cattggaaac tacgatggtg acgtttacaa gcattacttt 1980
   gccaaggtta accagcagaa ccctgctcag aacccccgac ctccttacta tgagagcact 2040
   cttcgacctt tcctgttccg agaggatgag gatgacgaca tttgcgagct ggacgaggaa 2100
   tag 2103
<210> 8
   <211> 2106
   <212> DNA
   <213> Yarrowia lipolytica
<220>
   <221> gene
   <222> (1)..(2106)
   <223> ACO4 gene
<400> 8
   atgatcaccc caaaccccgc taacgacatt gtccatgacg gcaagctcta cgacaccttc        60
   actgagcccc ccaagctgat ggctcaggag cgagctcagc tggacttcga ccctagagac       120
   atcacctact ttctggatgg ctctaaggag gagaccgagc tgctggagtc gctcatgctc       180
   atgtacgagc gagaccctct cttcaacaac cagaacgagt acgatgaatc gtttgaaaca       240
   ctgcgagagc gatctgtgaa gcgaattttc cagctgtcca agtccatcgc catggacccc       300
   gagcccatgt ctttccgaaa gattgggttc ctgggtattc ttgacatggg aacgtatgct       360
   cgactgggag tccactacgc gctcttctgt aactccatcc ggggccaggg aacccccgat       420
   cagctcatgt actggctgga ccagggagcc atggtcatca agggcttcta cggctgtttt       480
   gccatgaccg aaatgggcca tggatctaac ctgtcgcgtc tggaaaccat cgccactttc       540
   gacaaagaga ccgacgaatt tatcattaac acgccccacg ttggagccac aaagtggtgg       600
   attggtggtg ctgctcacac tgctactcac acacttgcct ttgcccgtct tcaagtagac       660
   ggaaaggact acggtgtgaa atcgtttgtc gtacctctcc gaaacctgga cgaccattcg       720
   ctgcgtcctg gaatcgccac aggtgatatt ggtaagaaga tgggtcgaga tgccgttgac 780
   aacggctgga ttcagttcac caacgtccga gtgccccgaa actacatgct catgaagcat 840
   accaaggttc ttcgagacgg taccgtcaag cagccgcctt tggcccaact gacttacgga 900
   tctctcatca ctggacgagt ccagatgacc actgactctc acaatgtgtc caaaaagttc 960
   ctcaccattg ccctgagata cgccaccatc cgacgacagt tctcgtcaac tccaggagag 1020
   cccgaaaccc gactaattga ctacctgtac caccaaagac gactcctgcc tcttatggct 1080
   tactcttacg ccatgaaact agctggagat cacgtccgag agctgttctt tgcatcccag 1140
   gagaaggctg agagcctcaa ggaggacgac aaagccggag ttgagtctta cgtccaggat 1200
   atcaaggagc tcttctctgt ttctgctggt ctcaaggctg ccactacatg ggcttgtgct 1260
   gacatcattg acaaggcccg acaggcgtgt ggaggccacg gatactctgc ctacaacggc 1320
   tttggacagg ccttccagga ctgggttgtc cagtgcactt gggagggtga caatactgtt 1380
   ctgactctat ctgccggccg agctctgatc caatctgctc tcgtctaccg aaaggagggc 1440
   aaactaggta acgccacgaa gtacctctct cggtccaagg agcttgccaa cgccaagaga 1500
   aacggacgat ccctggaaga ccccaagctg ctcgtggagg catgggaggc tgtctctgcc 1560
   ggtgctatca acgctgctac tgacgcttac gaggagctct ccaagcaggg agtttctgtt 1620
   gacgagtgct ttgagcaggt gtcccaggag cgattccagg ctgcccgaat ccacactcga 1680
   cgagctctta tcgaggcctt ctactcacga atcgccactg ctgatgagaa ggtgaagcct 1740
   catctgatcc ctctggccaa cctgtttgcc ctgtggtcca ttgaggagga ctctgctctg 1800
   ttcctggctg agggctactt tgagcctgag gatatcattg aggtgacttc tcttgtcaac 1860
   aagtactgcg gaattgttcg aaagaacgtt attggataca ccgatgcctt caacctgtcc 1920
   gactacttca tcaacgctgc cattggacga tacgacggag acgtgtacaa gaactacttt 1980
   gagaaggtca aacagcagta ccctcctgag ggtggcaagc ctcactacta cgaggatgtc 2040
   atgaagccct tcctgcatcg agagcgaatt cccgatgtcc ccatggagcc cgaggatatt 2100
   cagtaa 2106
<210> 9
   <211> 2100
   <212> DNA
   <213> Yarrowia lipolytica
<220>
   <221> gene
   <222> (1)..(2100)
   <223> ACO5 gene
<400> 9
<210> 10
   <211> 2070
   <212> DNA
   <213> Yarrowia lipolytica
<220>
   <221> gene
   <222> (1)..(2070)
   <223> ACO6 gene
<400> 10
   atgctctctc aacagtccct caacacgttt accgagcccc cggtcgaaat ggcccgggag      60
   cgaaaccaga cttccttcaa cccgcgtctg ctgacgtact ttctggacgg aggcgaaaag     120
   aacactctgc ttatggaccg actgatgcaa gagtacgagc gagaccctgt gtttcgaaac     180
   gagggcgact acgatattac cgatgtggcc cagtcgcgag agctggcctt caagcgaatc     240
   gccaagctca tcgagtatgt gcacaccgac gacgaggaga cgtatctgta ccgatgcatg     300
   cttctgggcc aaatcgatat gggagccttt gcccggtacg ccatccacca cggagtctgg     360
   ggcggtgcca ttcgaggtgc aggaacgcct gagcagtacg aattctgggt caagaaagga     420
   tctctgtcgg ttaagaagtt ctatggatcc ttctccatga ccgagctggg ccacggcagt     480
   aacttggtgg gtctggagac caccgccacc ctggacaaga acgcagacga gttcgtgatc     540
   aacactccca acgttgctgc cactaaatgg tggatcggag gagccgccga taccgccact     600
   cacacagctg tgtttgcacg tctcattgtc gacggagagg accacggtgt caagacgttt     660
   gtggtgcagc tgcgagacgt ggagactcac aacctgatgc ctggtattgc tatcggagac     720
   tgcggcaaga agatgggacg tcagggaacc gacaacggct ggatccagtt cacccatgtg     780
   cgaattcccc gacagaacat gctcatgcga tactgtcacg tggacagcga cggaaatgtt     840
   accgagccca tgatggctca gatggcctac ggagctcttc tggctggccg agtcggaatg     900
   gccatggaca gttatttcac ctcgcgaaag ttccttacca ttgctcttcg atatgccacc     960
   attcgacgag cttttgctgc cggaggaggt caggagacca agctgatcga ctacccttac    1020
   caccagcgac gtctgctccc cctcatggcc cagacatatg ccatcaagtg caccgccgat    1080
   aaggtcagag atcagttcgt caaggtcacc gacatgctcc taaacctcga tgtttctgac    1140
   caagaggccg tgcccaaggc cattgccgag gctaaggagc tcttctctgt ttctgctggt    1200
   gtcaaggcta ccacaacttg ggcttgcgca cacaccattg accagtgcag acaggcgtgt    1260
   ggaggccacg gatactctgc ttacaacggt tttggacgtg cttactccga ttgggtgatc 1320
   cagtgcacct gggagggaga caataacatt ctgtgtctgt cagctggcag agctctggtc 1380
   cagtctaacc gagctgtccg ggctggcaag cccattggag gtcctaccgc ctacctggct 1440
   gctcccgctg gttcccccaa gctcgctggt cgaaacttgt acgaccccaa ggtcatgatt 1500
   ggggcctggg agactgtttc ccgagctctg atcaaccgaa ccaccgatga gtttgaggtg 1560
   ctggccaaga agggtctgtc tactgcccag gcctacgagg agctgtccca gcaacgattc 1620
   ctgtgtactc gaatccacac ccgtctgtac atggtcaaga acttctacga gcgaattgcc 1680
   gaggagggca ccgagttcac caaggagcct cttaccagac ttgccaacct gtacgccttc 1740
   tggtccgtcg aagaggaggc tggaatcttc ctccgagagg gctacatcac tccccaggag 1800
   ctcaagtaca tcagtgccga gatccgaaag cagctcttgg aggtgcgaaa ggacgtcatt 1860
   ggctacaccg atgccttcaa cgtgcctgat tttttcctca actctgccat tggacgagct 1920
   gacggagatg tctacaagaa ctacttcaag gtggtcaaca ctcagaaccc tccccaagac 1980
   cctcgacctc cttattacga gtctgtcatt agacccttcc tgttccgaaa ggacgaggat 2040
   gaggaaattt gctctcttga ggatgagtag 2070
<210> 11
   <211> 1380
   <212> DNA
   <213> Chromobacterium violaceum DSM30191
<220>
   <221> gene
   <222> (1)..(1380)
   <223> transaminase gene
<400> 11
<210> 12
   <211> 1205
   <212> DNA
   <213> Yarrowia lipolytica
<220>
   <221> gene
   <222> (1)..(1205)
   <223> Ura3 gene
<400> 12
   cgcccagaga gccattgacg ttctttctaa tttggaccga tagccgtata gtccagtcta 60
   tctataagtt caactaactc gtaactatta ccataacata tacttcactg ccccagataa 120
   ggttccgata aaaagttctg cagactaaat ttatttcagt ctcctcttca ccaccaaaat 180
   gccctcctac gaagctcgag ctaacgtcca caagtccgcc tttgccgctc gagtgctcaa 240
   gctcgtggca gccaagaaaa ccaacctgtg tgcttctctg gatgttacca ccaccaagga 300
   gctcattgag cttgccgata aggtcggacc ttatgtgtgc atgatcaaaa cccatatcga 360
   catcattgac gacttcacct acgccggcac tgtgctcccc ctcaaggaac ttgctcttaa 420
   gcacggtttc ttcctgttcg aggacagaaa gttcgcagat attggcaaca ctgtcaagca 480
   ccagtaccgg tgtcaccgaa tcgccgagtg gtccgatatc accaacgccc acggtgtacc 540
   cggaaccgga atcattgctg gcctgcgagc tggtgccgag gaaactgtct ctgaacagaa 600
   gaaggaggac gtctctgact acgagaactc ccagtacaag gagttcctag tcccctctcc 660
   caacgagaag ctggccagag gtctgctcat gctggccgag ctgtcttgca agggctctct 720
   ggccactggc gagtactcca agcagaccat tgagcttgcc cgatccgacc ccgagtttgt 780
   ggttggcttc attgcccaga accgacctaa gggcgactct gaggactggc ttattctgac 840
   ccccggggtg ggtcttgacg acaagggaga cgctctcgga cagcagtacc gaactgttga 900
   ggatgtcatg tctaccggaa cggatatcat aattgtcggc cgaggtctgt acggccagaa 960
   ccgagatcct attgaggagg ccaagcgata ccagaaggct ggctgggagg cttaccagaa 1020
   gattaactgt tagaggttag actatggata tgtaatttaa ctgtgtatat agagagcgtg 1080
   caagtatgga gcgcttgttc agcttgtatg atggtcagac gacctgtctg atcgagtatg 1140
   tatgatactg cacaacctgt gtatccgcat gatctgtcca atggggcatg ttgttgtgtt 1200
   tctcg 1205
<210> 13
   <211> 36
   <212> DNA
   <213> Artificial Sequence
<220>
   <223> BglII F primer for **HisG1**
<400> 13
   aattgggccc agatctcaga ccggttcaga caggat 36
<210> 14
   <211> 36
   <212> DNA
   <213> Artificial Sequence
<220>
   <223> EcoRI R primer for HisG1
<400> 14
   tctctgggcg gaattcggag gtgcggatat gaggta 36
<210> 15
   <211> 37
   <212> DNA
   <213> Artificial Sequence
<220>
   <223> NotI F primer for HisG1
<400> 15
   tgtttctcgg cggccgccag accggttcag acaggat 37
<210> 16
   <211> 36
   <212> DNA
   <213> Artificial Sequence
<220>
   <223> BamHI R primer for HisG1
<400> 16
   tccaacgcgt ggatccggag gtgcggatat gaggta 36
<210> 17
   <211> 36
   <212> DNA
   <213> Artificial Sequence
<220>
   <223> BglII F primer for HisG2
<400> 17
   aattgggccc agatctaacg ctacctcgac cagaaa 36
<210> 18
   <211> 36
   <212> DNA
   <213> Artificial Sequence
<220>
   <223> EcoRI R primer for HisG2
<400> 18
   tctctgggcg gaattctctt ctcgatcggc agtacc 36
<210> 19
   <211> 37
   <212> DNA
   <213> Artificial Sequence
<220>
   <223> NotI F primer for HisG2
<400> 19
   tgtttctcgg cggccgcaac gctacctcga ccagaaa 37
<210> 20
   <211> 36
   <212> DNA
   <213> Artificial Sequence
<220>
   <223> BamHI R primer for HisG2
<400> 20
   tccaacgcgt ggatcctctt ctcgatcggc agtacc 36
<210> 21
   <211> 36
   <212> DNA
   <213> Artificial Sequence
<220>
   <223> BglII F primer for glt2
<400> 21
   aattgggccc agatcttcag aacttgcgcc gataaa 36
<210> 22
   <211> 38
   <212> DNA
   <213> Artificial Sequence
<220>
   <223> EcoRI R primer for glt2
<400> 22
   tctctgggcg gaattccttt gccagctaga ccatagag 38
<210> 23
   <211> 37
   <212> DNA
   <213> Artificial Sequence
<220>
   <223> NotI F primer for glt2
<400> 23
   tgtttctcgg cggccgctca gaacttgcgc cgataaa 37
<210> 24
   <211> 38
   <212> DNA
   <213> Artificial Sequence
<220>
   <223> BamHI R primer for glt2
<400> 24
   tccaacgcgt ggatcccttt gccagctaga ccatagag 38
<210> 25
   <211> 35
   <212> DNA
   <213> Artificial Sequence
<220>
   <223> BglII F primer for glt3
<400> 25
   aattgggccc agatctattg gcgggttcgt tactt 35
<210> 26
   <211> 38
   <212> DNA
   <213> Artificial Sequence
<220>
   <223> EcoRI R primer for glt3
<400> 26
   tctctgggcg gaattccctg gaagaaggcc gtattatc 38
<210> 27
   <211> 36
   <212> DNA
   <213> Artificial Sequence
<220>
   <223> NotI F primer for glt3
<400> 27
   tgtttctcgg cggccgcatt ggcgggttcg ttactt 36
<210> 28
   <211> 38
   <212> DNA
   <213> Artificial Sequence
<220>
   <223> BamHI R primer for glt3
<400> 28
   tccaacgcgt ggatcccctg gaagaaggcc gtattatc 38
<210> 29
   <211> 20
   <212> DNA
   <213> Artificial Sequence
<220>
   <223> F1 primer for ACO1
<400> 29
   ttcctcaatg gtggagaaga 20
<210> 30
   <211> 46
   <212> DNA
   <213> Artificial Sequence
<220>
   <223> R1 primer for ACO1
<400> 30
   tctttatcct gtctgaaccg gtctggtacc atagtccttg ccatgc 46
<210> 31
   <211> 45
   <212> DNA
   <213> Artificial Sequence
<220>
   <223> F2 primer for ACO1
<400> 31
   atcgctacct catatccgca cctcccttct gtcccccgag tttct 45
<210> 32
   <211> 19
   <212> DNA
   <213> Artificial Sequence
<220>
   <223> R2 primer for ACO1
<400> 32
   aagaagggct tgagagtcg 19
<210> 33
   <211> 17
   <212> DNA
   <213> Artificial Sequence
<220>
   <223> F1 primer for ACO2
<400> 33
   cccaacaaca ctggcac 17
<210> 34
   <211> 43
   <212> DNA
   <213> Artificial Sequence
<220>
   <223> R1 primer for ACO2
<400> 34
   tctttatcct gtctgaaccg gtctgctcct catcgtagat ggc 43
<210> 35
   <211> 42
   <212> DNA
   <213> Artificial Sequence
<220>
   <223> F2 primer for ACO2
<400> 35
   atcgctacct catatccgca cctccgacaa gacccgacag gc 42
<210> 36
   <211> 18
   <212> DNA
   <213> Artificial Sequence
<220>
   <223> R2 primer for ACO2
<400> 36
   agaccagagt cctcttcg 18
<210> 37
   <211> 19
   <212> DNA
   <213> Artificial Sequence
<220>
   <223> F1 primer for ACO3
<400> 37
   accttcacag agccaccca 19
<210> 38
   <211> 36
   <212> DNA
   <213> Artificial Sequence
<220>
   <223> R1 primer for ACO3
<400> 38
   atggctctct gggcggtgtt gggggtgttg atgatg 36
<210> 39
   <211> 36
   <212> DNA
   <213> Artificial Sequence
<220>
   <223> F2 primer for ACO3
<400> 39
   ttgttgtgtt tctcgcaagg ttctcatcga ggcctg 36
<210> 40
   <211> 22
   <212> DNA
   <213> Artificial Sequence
<220>
   <223> R2 primer for ACO3
<400> 40
   aggaaaggtc gaagagtgct ct 22
<210> 41
   <211> 17
   <212> DNA
   <213> Artificial Sequence
<220>
   <223> F1 primer for ACO4
<400> 41
   actgcgagag cgatctg 17
<210> 42
   <211> 45
   <212> DNA
   <213> Artificial Sequence
<220>
   <223> R1 primer for ACO4
<400> 42
   tctttatcct gtctgaaccg gtctgttcat gagcatgtag tttcg 45
<210> 43
   <211> 44
   <212> DNA
   <213> Artificial Sequence
<220>
   <223> F2 primer for ACO4
<400> 43
   atcgctacct catatccgca cctccgagga cgacaaagcc ggag 44
<210> 44
   <211> 19
   <212> DNA
   <213> Artificial Sequence
<220>
   <223> R2 primer for ACO4
<400> 44
   agagcagagt cctcctcaa
<210> 45
   <211> 22
   <212> DNA
   <213> Artificial Sequence
<220>
   <223> F1 primer for ACO5
<400> 45
   aacttcctca caggcagcga gc 22
<210> 46
   <211> 39
   <212> DNA
   <213> Artificial Sequence
<220>
   <223> R1 primer for ACO5
<400> 46
   atggctctct gggcggagta gagagtggga gttgaggtc 39
<210> 47
   <211> 34
   <212> DNA
   <213> Artificial Sequence
<220>
   <223> F2 primer for ACO5
<400> 47
   ttgttgtgtt tctcgccccg tcaaggacgc tgag 34
<210> 48
   <211> 22
   <212> DNA
   <213> Artificial Sequence
<220>
   <223> R2 primer for ACO5
<400> 48
   acagtaaggt ggggcttgac tc 22
<210> 49
   <211> 21
   <212> DNA
   <213> Artificial Sequence
<220>
   <223> F1 primer for ACO6
<400> 49
   agtccctcaa cacgtttacc g 21
<210> 50
   <211> 46
   <212> DNA
   <213> Artificial Sequence
<220>
   <223> R1 primer for ACO6
<400> 50
   tctttatcct gtctgaaccg gtctgccatt tagtggcagc aacgtt 46
<210> 51
   <211> 45
   <212> DNA
   <213> Artificial Sequence
<220>
   <223> F2 primer for ACO6
<400> 51
   atcgctacct catatccgca cctccgagct ctgatcaacc gaacc 45
<210> 52
   <211> 20
   <212> DNA
   <213> Artificial Sequence
<220>
   <223> R2 primer for ACO6
<400> 52
   aggaagggtc taatgacaga 20
<210> 53
   <211> 17
   <212> DNA
   <213> Artificial Sequence
<220>
   <223> F1 primer for FALDH1
<400> 53
   aatcactcct cctacgc 17
<210> 54
   <211> 43
   <212> DNA
   <213> Artificial Sequence
<220>
   <223> R1 primer for FALDH1
<400> 54
   tctttatcct gtctgaaccg gtctgtggtc tcggggacac ctc 43
<210> 55
   <211> 43
   <212> DNA
   <213> Artificial Sequence
<220>
   <223> F2 primer for FALDH1
<400> 55
   atcgctacct catatccgca cctccccatc atcaagcccc gaa 43
<210> 56
   <211> 20
   <212> DNA
   <213> Artificial Sequence
<220>
   <223> R2 primer for FALDH1
<400> 56
   accgacataa tctgagcaat 20
<210> 57
   <211> 19
   <212> DNA
   <213> Artificial Sequence
<220>
   <223> F1 primer for FALDH2
<400> 57
   accactaggt gagatcgag 19
<210> 58
   <211> 45
   <212> DNA
   <213> Artificial Sequence
<220>
   <223> R1 primer for FALDH2
<400> 58
   tctttatcct gtctgaaccg gtctgctccg acactaccgg aacgc 45
<210> 59
   <211> 43
   <212> DNA
   <213> Artificial Sequence
<220>
   <223> F2 primer for FALDH2
<400> 59
   atcgctacct catatccgca cctcccttgc tcccacagtt gtt 43
<210> 60
   <211> 19
   <212> DNA
   <213> Artificial Sequence
<220>
   <223> R2 primer for FALDH2
<400> 60
   gatcacccag aaccatagc 19
<210> 61
   <211> 19
   <212> DNA
   <213> Artificial Sequence
<220>
   <223> F1 primer for FALDH3
<400> 61
   gtgaccccca ccacgtcac 19
<210> 62
   <211> 46
   <212> DNA
   <213> Artificial Sequence
<220>
   <223> R1 primer for FALDH3
<400> 62
   tctttatcct gtctgaaccg gtctgttctg acattttcag cgccac 46
<210> 63
   <211> 45
   <212> DNA
   <213> Artificial Sequence
<220>
   <223> F2 primer for FALDH3
<400> 63
   atcgctacct catatccgca cctccccatt acgagcgttt gacgg 45
<210> 64
   <211> 17
   <212> DNA
   <213> Artificial Sequence
<220>
   <223> R2 primer for FALDH3
<400> 64
   cagggctggg gaccacc 17
<210> 65
   <211> 19
   <212> DNA
   <213> Artificial Sequence
<220>
   <223> F1 primer for FALDH4
<400> 65
   taccgactgg accagattc 19
<210> 66
   <211> 46
   <212> DNA
   <213> Artificial Sequence
<220>
   <223> R1 primer for FALDH4
<400> 66
   tctttatcct gtctgaaccg gtctgcggca gtggcaatga tcttac 46
<210> 67
   <211> 46
   <212> DNA
   <213> Artificial Sequence
<220>
   <223> F2 primer for FALDH4
<400> 67
   atcgctacct catatccgca cctccgactc gattcatcgc tcctac 46
<210> 68
   <211> 21
   <212> DNA
   <213> Artificial Sequence
<220>
   <223> R2 primer for FALDH4
<400> 68
   caaatctttc ggaagattcg g 21
<210> 69
   <211> 20
   <212> DNA
   <213> Artificial Sequence
<220>
   <223> F primer for HISG1
<400> 69
   cagaccggtt cagacaggat 20
<210> 70
   <211> 20
   <212> DNA
   <213> Artificial Sequence
<220>
   <223> R primer for HISG1
<400> 70
   ggaggtgcgg atatgaggta 20
<210> 71
   <211> 20
   <212> DNA
   <213> Artificial Sequence
<220>
   <223> F primer for HISG2
<400> 71
   aacgctacct cgaccagaaa 20
<210> 72
   <211> 20
   <212> DNA
   <213> Artificial Sequence
<220>
   <223> R primer for HISG2
<400> 72
   tcttctcgat cggcagtacc 20
<210> 73
   <211> 20
   <212> DNA
   <213> Artificial Sequence
<220>
   <223> F primer for glt2
<400> 73
   tcagaacttg cgccgataaa 20
<210> 74
   <211> 22
   <212> DNA
   <213> Artificial Sequence
<220>
   <223> R primer for glt2
<400> 74
   ctttgccagc tagaccatag ag 22
<210> 75
   <211> 19
   <212> DNA
   <213> Artificial Sequence
<220>
   <223> F primer for glt3
<400> 75
   attggcgggt tcgttactt 19
<210> 76
   <211> 22
   <212> DNA
   <213> Artificial Sequence
<220>
   <223> R primer for glt3
<400> 76
   cctggaagaa ggccgtatta tc 22
<210> 77
   <211> 23
   <212> DNA
   <213> Artificial Sequence
<220>
   <223> Ulura3 cs 2B primer for Bipartite
<400> 77
   atgccctcct acgaagctcg agc 23
<210> 78
   <211> 20
   <212> DNA
   <213> Artificial Sequence
<220>
   <223> Ylura3F primer for Bipartite
<400> 78
   ctcccaacga gaagctggcc 20
<210> 79
   <211> 42
   <212> DNA
   <213> Artificial Sequence
<220>
   <223> EXP1-F primer for transaminase vector
<400> 79
   ccaagcttgg taccgagctc agagtttggc gcccgttttt tc 42
<210> 80
   <211> 42
   <212> DNA
   <213> Artificial Sequence
<220>
   <223> EXP1-R primer for transaminase vector
<400> 80
   cgttgttttt gcatatgtgc tgtagatatg tcttgtgtgt aa 42
<210> 81
   <211> 42
   <212> DNA
   <213> Artificial Sequence
<220>
   <223> TEF-F primer for transaminase vector
<400> 81
   ccaagcttgg taccgagctc aaactttggc aaagaggctg ca 42
<210> 82
   <211> 42
   <212> DNA
   <213> Artificial Sequence
<220>
   <223> TEF-R primer for transaminase vector
<400> 82
   cgttgttttt gcatatgttt gaatgattct tatactcaga ag 42
<210> 83
   <211> 44
   <212> DNA
   <213> Artificial Sequence
<220>
   <223> ALK1-F primer for transaminase vector
<400> 83
   ccaagcttgg taccgagctc agatctgtgc gcctctacag accc 44
<210> 84
   <211> 41
   <212> DNA
   <213> Artificial Sequence
<220>
   <223> ALK1-R primer for transaminase vector
<400> 84
   cgttgttttt gcatatgagt gcaggagtat tctggggagg a 41
<210> 85
   <211> 28
   <212> DNA
   <213> Artificial Sequence
<220>
   <223> XPR2t-F2 primer for transaminase vector
<400> 85
   gtcgacgcaa ttaacagata gtttgccg 28
<210> 86
   <211> 32
   <212> DNA
   <213> Artificial Sequence
<220>
   <223> XPR2t-R3 primer for transaminase vector
<400> 86
   ctcgagggat cccggaaaac aaaacacgac ag 32
<210> 87
   <211> 28
   <212> DNA
   <213> Artificial Sequence
<220>
   <223> TA-F primer for transaminase vector
<400> 87
   catatgcaaa aacaacgtac tacctccc 28
<210> 88
   <211> 28
   <212> DNA
   <213> Artificial Sequence
<220>
   <223> TA-R primer for transaminase vector
<400> 88
   gtcgacttag gccaaaccac gggctttc 28
<210> 89
   <211> 35
   <212> DNA
   <213> Artificial Sequence
<220>
   <223> ATATG2-ER-F primer for transaminase vector
<400> 89
   actcctgcac tcatatgtcc aacgccctca acctg 35
<210> 90
   <211> 35
   <212> DNA
   <213> Artificial Sequence
<220>
   <223> XTATG2-ER-F primer for transaminase vector
<400> 90
   ccaatccaac acatatgtcc aacgccctca acctg 35
<210> 91
   <211> 82
   <212> DNA
   <213> Artificial Sequence
<220>
   <223> ER-R-1 primer for transaminase vector
<400> 91
   cgttgttttt gcatagaacc gccaccgccg ctaccgccac cgcccgaacc gccaccgccg 60
aatcgtgaaa tatccttggg ct 82
<210> 92
   <211> 85
   <212> DNA
   <213> Artificial Sequence
<220>
   <223> ER-R-2 primer for transaminase vector
<400> 92
   cgttgttttt gcatatgaga accgccaccg ccgctaccgc caccgcccga accgccaccg 60
ccgaatcgtg aaatatcctt gggct 85
<210> 93
   <211> 37
   <212> DNA
   <213> Artificial Sequence
<220>
   <223> ETATG2-ER-1 primer for transaminase vector
<400> 93
   tgattacgcc aagcttgagt ttggcgcccg ttttttc 37
<210> 94
   <211> 50
   <212> DNA
   <213> Artificial Sequence
<220>
   <223> ETATG2-ER-2 primer for transaminase vector
<400> 94
   acaggttgag ggcgttggac atatgtgctg tagatatgtc ttgtgtgtaa 50
<210> 95
   <211> 36
   <212> DNA
   <213> Artificial Sequence
<220>
   <223> TTATG2-ER-1 primer for transaminase vector
<400> 95
   tgattacgcc aagcttaaac tttggcaaag aggctg 36
<210> 96
   <211> 50
   <212> DNA
   <213> Artificial Sequence
<220>
   <223> TTATG2-ER-2 primer for transaminase vector
<400> 96
   acaggttgag ggcgttggac atatgtttga atgattctta tactcagaag 50
<210> 97
   <211> 21
   <212> DNA
   <213> Artificial Sequence
<220>
   <223> ER-F primer for transaminase vector
<400> 97
   atgtccaacg ccctcaacct g 21
<210> 98
   <211> 34
   <212> DNA
   <213> Artificial Sequence
<220>
   <223> ER-R-3 primer for transaminase vector
<400> 98
   cgttgttttt gcatagaacc gccaccgccg ctac 34
<210> 99
   <211> 19
   <212> DNA
   <213> Artificial Sequence
<220>
   <223> TA-FALDH4-F1 primer for transaminase vector
<400> 99
   taccgactgg accagattc 19
<210> 100
   <211> 21
   <212> DNA
   <213> Artificial Sequence
<220>
   <223> TA-FALDH4-R1 primer for transaminase vector
<400> 100
   cggcagtggc aatgatctta c 21
<210> 101
   <211> 45
   <212> DNA
   <213> Artificial Sequence
<220>
   <223> TA-FALDH4-F2 primer for transaminase vector
<400> 101
   ctcctctatg gtctagctgg caaagactcg attcatcgct cctac 45
<210> 102
   <211> 21
   <212> DNA
   <213> Artificial Sequence
<220>
   <223> TA-FALDH4-R2 primer for transaminase vector
<400> 102
   caaatctttc ggaagattcg g 21
<210> 103
   <211> 47
   <212> DNA
   <213> Artificial Sequence
<220>
   <223> ATATG2-F primer for transaminase vector
<400> 103
   gtcggtaaga tcattgccac tgccgagatc tgtgcgcctc tacagac 47
<210> 104
   <211> 46
   <212> DNA
   <213> Artificial Sequence
<220>
   <223> ETATG2-F primer for transaminase vector
<400> 104
   gtcggtaaga tcattgccac tgccggagtt tggcgcccgt tttttc 46
<210> 105
   <211> 46
   <212> DNA
   <213> Artificial Sequence
<220>
   <223> TTATG2-F primer for transaminase vector
<400> 105
   gtcggtaaga tcattgccac tgccgaaact ttggcaaaga ggctgc 46
<210> 106
   <211> 45
   <212> DNA
   <213> Artificial Sequence
<220>
   <223> XTATG2-F primer for transaminase vector
<400> 106
   gtcggtaaga tcattgccac tgccgacgcg tggagagttt gggtt 45

## Description

### [Technical Field]

The present invention relates to a method for producing a medium chain aminocarboxylic acid, and more particularly, to a method for producing a medium chain aminocarboxylic acid from a fatty acid by culturing a recombinant microorganism from which a fatty aldehyde dehydrogenase (or fatty alcohol dehydrogenase) gene in an ω-oxidative metabolism pathway and β-oxidative metabolism pathway-related genes are deleted and into which an ω-transaminase gene is also introduced.

### [Background Art]

Bioplatform compounds are produced through biological or chemical conversion on the basis of biomass-derived raw materials, and thus have been used for synthesis of polymeric monomers, new materials, and the like.

Among the bioplatform compounds, a medium chain aminocarboxylic acid is a material used as a monomer for polyamides. The polyamides are classified into aliphatic polyamides, aromatic polyamides, and aliphatic cyclic polyamides. Representative examples of the aliphatic polyamides includes Nylon 12, Nylon 6, and Nylon 66, and the aromatic polyamides have an aromatic framework introduced therein in order to further improve heat resistance, and are also known under the name of aramid.

Since the 1940's, nylon is a representative engineering plastic material whose demand and use have increased steadily due to high crystallinity, mechanical strength and thermal stability, excellent wear/friction resistance characteristics, and the like. Also, there has been continuous research conducted in various fields to improve the thermal and mechanical properties of nylon. Among theses, there is research conducted to impregnate wax and graphite so as to improve wear resistance of nylon. In addition, there is ongoing research conducted to improve the physical properties of nylon through the crosslinking of polymers. Among the types of nylon, Nylon 12 synthesized through the polycondensation of 12-aminododecanoic acid exhibits low specific gravity, excellent low-temperature characteristics and wear resistance and high weather resistance due to the insignificant effects of ultraviolet rays, and also has a very short -CH₂- chain length, compared to other nylon resins (i.e., Nylons 6, 66). Therefore, because Nylon 12 has a low probability of having a hydrogen bond of H₂O to an amide functional group when present in the same weight, Nylon 12 may serve to prevent the degradation of mechanical strength caused by moisture absorption, which is one of the biggest drawbacks of the nylon resins, which makes it possible to widely apply it to materials for automobile parts, aircraft materials, heat-resistant special fibers, and the like (Beomsik, Shin, et al., Polymer, 35(1):30-34, 2011).

Production of medium chain aminocarboxylic acids such as 12-aminododecanoic acid may be carried out using biological methods through chemical synthesis or microbial fermentation. In this case, the use of such biological methods requires the development of novel strains and the optimization of fermentation processes using metabolic engineering technology.

In the prior art, a microorganism which harbors both a β-oxidative metabolism pathway and an ω-oxidative metabolism pathway may be used as the strain capable of producing a medium chain aminocarboxylic acid. For example, a method for producing ω-aminododecanoic acid in *Escherichia coli* is known (US 2010/0324257 A1). However, because the medium chain aminocarboxylic acid is prepared by further introducing a process of transferring an amine group to a medium chain aldehyde carboxylic acid, the medium chain aminocarboxylic acid has a drawback in that it may not be produced with high yield when it is produced using the microorganism.

### [Disclosure]

### [Technical Problem]

Therefore, it is an object of the present invention to provide a recombinant microorganism from which a fatty aldehyde dehydrogenase gene in an ω-oxidative metabolism pathway and β-oxidative metabolism pathway-related genes are deleted and into which an ω-transaminase gene is also introduced, and a method for producing a medium chain aminocarboxylic acid from a fatty acid by culturing the recombinant microorganism.

### [Technical Solution]

To solve the above problems, according to an aspect of the present invention, there is provided a recombinant microorganism from which a fatty aldehyde dehydrogenase gene in an ω-oxidative metabolism pathway and β-oxidative metabolism pathway-related genes are deleted and into which an ω-transaminase gene is also introduced.

According to an embodiment of the present invention, the fatty aldehyde dehydrogenase gene and the β-oxidative metabolism pathway-related genes are preferably deleted from all homologous genes present in the microorganism, but the present invention is not limited thereto. According to another embodiment of the present invention, the fatty aldehyde dehydrogenase gene and the β-oxidative metabolism pathway-related genes are preferably deleted from some of the homologous genes present in the corresponding microorganism, but the present invention is not limited thereto.

According to an embodiment of the present invention, the fatty aldehyde dehydrogenase gene may be a gene selected from the group consisting of FALDH1, FALDH2, FALDH3, and FALDH4 genes, but the present invention is not limited thereto.

According to an embodiment of the present invention, the β-oxidative metabolism pathway-related genes may be an acyl-CoA oxidase gene, but the present invention is not limited thereto. According to preferred embodiments of the present invention, the acyl-CoA oxidase gene may be selected from the group consisting of ACO1, ACO2, ACO3, ACO4, ACO5, and ACO6 genes, but the present invention is not limited thereto.

According to an embodiment of the present invention, the microorganism may be a yeast or *Escherichia coli,* but the present invention is not limited thereto. According to preferred embodiments of the present invention, the yeast may be selected from the group of the yeast consisting of *Yarrowia* sp., *Saccharomyces* sp., *Pichia* sp., and *Candida* sp., but the present invention is not limited thereto. According to other preferred embodiments of the present invention, the Yarrowia sp. yeast may be *Yarrowia lipolytica,* but the present invention is not limited thereto.

According to another aspect of the present invention, there is provided a method for producing a medium chain aminocarboxylic acid, which comprises (1) preparing the recombinant microorganism from which a fatty aldehyde dehydrogenase gene in an ω-oxidative metabolism pathway and β-oxidative metabolism pathway-related genes are deleted and into which an ω-transaminase gene is also introduced; and (2) treating the recombinant microorganism with a substrate to culture the recombinant microorganism.

According to an embodiment of the present invention, the substrate may include a fatty acid, but the present invention is not limited thereto. According to preferred embodiments of the present invention, the fatty acid and medium chain aminocarboxylic acid may have 5 to 30 carbon atoms, preferably 6 to 20 carbon atoms, and more preferably 8 to 16 carbon atoms, but the present invention is not limited thereto. According to other preferred embodiments of the present invention, the fatty acid may be dodecanoic acid, but the present invention is not limited thereto. According to other preferred embodiments of the present invention, the medium chain aminocarboxylic acid may be 12-aminododecanoic acid, but the present invention is not limited thereto.

### [Advantageous Effects]

A recombinant microorganism of the present invention can produce a medium chain aminocarboxylic acid, for example, 12-aminodecane used as a raw material of Nylon 12, from a substrate such as a fatty acid by deleting a fatty aldehyde dehydrogenase gene in an ω-oxidative metabolism pathway and β-oxidative metabolism pathway-related genes and introducing an ω-transaminase gene.

### [Description of Drawings]

FIG. 1 is a diagram showing types of products and related enzymes associated with ω-oxidative and β-oxidative metabolism reactions.
FIG. 2 is a diagram schematically showing a process of preparing a recombinant microorganism of the present invention from which a fatty aldehyde dehydrogenase gene associated with ω-oxidation and β-oxidative metabolism pathway-related genes are deleted and into which an ω-transaminase gene is also introduced.
FIG. 3 is a diagram schematically showing a vector containing an ura3 gene to be used as a selective marker for gene knockout to modify a strain, and a pop-out region for deleting the ura3 gene after insertion of a knock-out cassette.
FIG. 4 is a schematic diagram showing a process of constructing a knock-out cassette used to prepare a transformant microorganism according to the present invention.
FIG. 5 is a diagram schematically showing a transformation vector containing an ω-transaminase gene for the purpose of modifying a strain.
FIG. 6 is a graph illustrating types of transduced knock-out genes in the transformant microorganism according to the present invention.
FIG. 7 is a graph illustrating an amount of a medium chain aminocarboxylic acid produced from the dodecanoic acid substrate, using the transformant microorganism according to the present invention.
FIG. 8 is a graph illustrating an amount of the medium chain aminocarboxylic acid produced from the dodecanoic acid substrate, when an Y2-36 strain of the present invention is cultured in a flask.
FIG. 9 shows the GC/MS data showing that the medium chain aminocarboxylic acid is produced from the dodecanoic acid substrate in the Y2-36 strain according to the present invention.

### [Best Mode]

To achieve the objectives of the present invention, the present invention provides a recombinant microorganism from which a fatty aldehyde dehydrogenase gene in an ω-oxidative metabolism pathway and β-oxidative metabolism pathway-related genes are deleted and into which an ω-transaminase gene is also introduced.

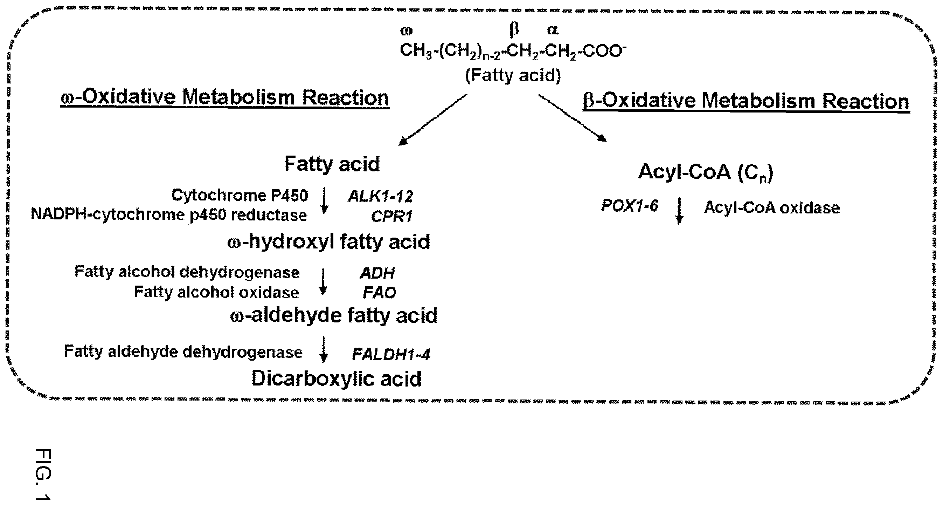

In the present invention, the term "ω-oxidation" refers to a metabolic process in which the terminal methyl group of a fatty acid is oxidized to form dicarboxylic acid, and the term "β-oxidation" refers to a metabolic process in which a carbon atom at the β-position in a carboxyl group is oxidized to release acetyl-CoA, whereby fatty acids are gradually decomposed into fatty acids whose number of carbon atoms is reduced by two. The concept of the ω- and β-oxidations and the enzymes involved in such metabolic processes are widely known to persons having ordinary skill in the field of biochemistry. For example, when a fatty acid is used as the substrate for ω-oxidation, an ω-hydroxy fatty acid is first produced by means of an action of cytochrome P450 and an NADPH-cytochrome P450 reductase. Then, the ω-hydroxy fatty acid is converted into ω-aldehyde fatty acid by an action of a fatty alcohol dehydrogenase and a fatty alcohol oxidase, and the ω-aldehyde fatty acid is converted into dicarboxylic acid by an action of a fatty aldehyde dehydrogenase. Also, for the β-oxidation, a fatty acid whose number of carbon atoms is reduced by two is produced by an acyl-CoA oxidase (see FIG. 1).

Transaminase (TA, EC 2.6.1.X) is an enzyme which exists widely in nature and is involved in the transfer of an amine group in the nitrogen metabolism of an organism. Generally, transaminases serve to remove an amino group from one amino acid to transfer the amino group to another α-keto acid. The transaminases are used to produce optically pure non-natural amino acids and amine compounds because the transaminases have various outstanding advantages in that they exhibit wide specificity to substrates, high optical selectivity, a rapid reaction rate, and superior stability, and have no need for reproduction of coenzymes, and the like. The transaminases may be classified into five groups depending on the structures and multisequence alignments of proteins found in the Pfam database. Among these, the transaminases belonging to Group III including an ω-amino acid:pyruvate transaminase, an ornithine transaminase, a 4-aminobutyrate transaminase, and the like are referred to as ω-transaminases. Unlike the typical transaminases, the ω-transaminases perform a reaction of transferring an amine group of an amino acid- or carboxyl group-free amine compound, which contains an amine group at a position other than the α-position, to an amine receptor such as 2-ketoglutarate or pyruvate. Therefore, the ω-transaminases may be used as enzymes very useful for production of optically active amine compounds. For example, the ω-transaminases were first employed at 1990 by Celgene Co. (USA) to synthesize chiral amines. In recent years, the ω-transaminases have been importantly employed for studies on asymmetric synthesis of chiral amines and studies on improvement of kinetic resolution. In 2012, Evonik Industries AG (Germany) reported one case in which 12-oxolauric acid methyl ester is converted into 12-aminolauric acid methyl ester using an ω-transaminase of a *Chromobacterium violaceum* DSM30191 strain.

According to an embodiment of the present invention, the fatty aldehyde dehydrogenase gene is preferably deleted from all homologous genes present in the corresponding microorganism, but a recombinant microorganism from which some of these genes are deleted may also be applied to the present invention, when necessary.

According to an embodiment of the present invention, the fatty aldehyde dehydrogenase gene may be selected from the group consisting of FALDH1, FALDH2, FALDH3, and FALDH4 genes, but the present invention is not limited thereto. The FALDH1, FALDH2, FALDH3, and FALDH4 genes may comprise base sequences set forth in SEQ ID NOs: 1 to 4, respectively, but the present invention is not limited thereto.

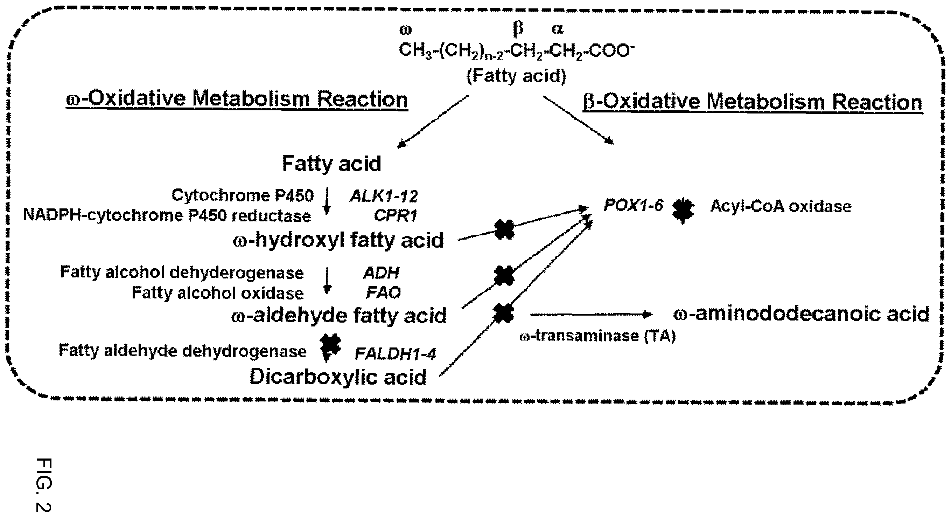

According to another embodiment of the present invention, the β-oxidative metabolism pathway-related genes are preferably deleted from all homologous genes present in the corresponding microorganism, but a recombinant microorganism from which some of these genes are deleted may also be applied to the present invention, when necessary. The β-oxidative metabolism pathway-related genes preferably includes an acyl-CoA oxidase gene, and the acyl-CoA oxidase gene may be selected from the group consisting of ACO1, ACO2, ACO3, ACO4, ACO5, and ACO6 genes, but the present invention is not limited thereto (see FIG. 2). According to other preferred embodiments of the present invention, the ACO1, ACO2, ACO3, ACO4, ACO5, and ACO6 genes may comprise base sequences set forth in SEQ ID NOs: 5 to 10, respectively, but the present invention is not limited thereto.

According to another embodiment of the present invention, the ω-transaminase gene may comprise a base sequence set forth in SEQ ID NO: 11, but the present invention is not limited thereto.

In the present invention, the recombinant microorganism from which the fatty aldehyde dehydrogenase gene and the β-oxidative metabolism pathway-related genes are deleted and into which the ω-transaminase gene is also introduced may be prepared using conventional genetic recombinant technology known in the related art. In the present invention, the term "deletion" is used as a meaning generally encompassing a physical deletion of part or all of the corresponding gene, and also encompassing a situation in which a protein is not expressed from mRNA transcribed from the corresponding gene and a situation in which a protein expressed from the corresponding gene does not function. Also, the term "introduction" is used as a meaning generally encompassing all situations in which a gene is inserted into the genome of a microorganism, or a gene is expressed without insertion of the corresponding gene into the genome of the microorganism. Examples of the genetic recombinant technology that may be used herein may include methods such as transformation, transduction, transfection, microinjection, electroporation, and the like, but the present invention is not limited thereto.

In the present invention, any microorganisms having both ω-oxidative and β-oxidative metabolism processes may be used without limitation. For example, eukaryotes including a yeast and prokaryotes including *Escherichia coli* may be used. According to an embodiment of the present invention, the yeast is preferably used as the microorganism. In this case, yeasts such as *Yarrowia* sp., *Saccharomyces* sp., *Pichia* sp., *Candida* sp., and the like may be used as the yeast without limitation. Among theses, *Yarrowia lipolytica, Candida tropicalis, Candida infanticola, Saccharomyces cerevisiae, Pichia alcoholophia,* or *Candida mycoderma* is preferably used. *Yarrowia lipolytica* is more preferably used.

As described above, in the case of the microorganism from which the fatty aldehyde dehydrogenase gene and the β-oxidative metabolism pathway-related gene are deleted and into which the ω-transaminase gene is also introduced, when a fatty acid is supplied as the substrate, an opposite terminal of a carboxyl group is oxidized by an action of cytochrome P450 and an NADPH-cytochrome P450 reductase to form an alcohol. Then, a hydroxyl group of the alcohol is oxidized by an action of a fatty alcohol dehydrogenase and a fatty alcohol oxidase to form an aldehyde. However, because the fatty aldehyde dehydrogenase is deletion, no further oxidation occurs anymore. Also, the fatty acid aldehyde thus formed is aminated by an action of an ω-transaminase to form an aminocarboxylic acid.

Also, the present invention provides a method for producing a medium chain aminocarboxylic acid, which comprises:
(1) preparing a recombinant microorganism from which a fatty aldehyde dehydrogenase gene in an ω-oxidative metabolism pathway and β-oxidative metabolism pathway-related genes are deleted and into which an ω-transaminase gene is also introduced; and
(2) treating the recombinant microorganism with a substrate to culture the recombinant microorganism.

In the present invention, the recombinant microorganism, from which the fatty aldehyde dehydrogenase gene in the ω-oxidative metabolism pathway and the β-oxidative metabolism pathway-related genes are deleted and into which the ω-transaminase gene is also introduced, may be used to produce a medium chain aminocarboxylic acid with high yield by preventing additional oxidation and β-oxidative metabolism of fatty acid aldehydes and introducing an amine group to the medium chain aldehyde fatty acid as well. The fatty aldehyde dehydrogenase gene and the β-oxidative metabolism pathway-related genes are preferably deleted from all homologous genes present in the corresponding microorganism, but a recombinant microorganism from which some of these genes are deleted may also be applied to the present invention, when necessary.

In the present invention, any microorganisms having both ω-oxidative and β-oxidative metabolism processes may be used without limitation. For example, eukaryotes including a yeast and prokaryotes including *Escherichia coli* may be used. According to an embodiment of the present invention, the yeast is preferably used as the microorganism. In this case, yeasts such as *Yarrowia* sp., *Saccharomyces* sp., *Pichia* sp., *Candida* sp., and the like may be used as the yeast without limitation. Among theses, *Yarrowia lipolytica, Candida tropicalis, Candida infanticola, Saccharomyces cerevisiae, Pichia alcoholophia,* or *Candida mycoderma* is preferably used. *Yarrowia lipolytica* is more preferably used.

In the present invention, the recombinant microorganism from which the fatty aldehyde dehydrogenase gene and the β-oxidative metabolism pathway-related genes are deleted and into which the ω-transaminase gene is also introduced may be prepared using conventional genetic recombinant technology known in the related art. In the present invention, the term "deletion" is used as a meaning generally encompassing a physical deletion of part or all of the corresponding gene, and also encompassing a situation in which a protein is not expressed from mRNA transcribed from the corresponding gene and a situation in which a protein expressed from the corresponding gene does not function. Also, the term "introduction" is used as a meaning generally encompassing all situations in which a gene is inserted into the genome of a microorganism, or a gene is expressed without insertion of the corresponding gene into the genome of the microorganism.

In the present invention, the "medium chain aminocarboxylic acid" is used as a meaning encompassing all medium chain aminocarboxylic acids having 5 to 30 carbon atoms, preferably 8 to 16 carbon atoms. According to preferred embodiments of the present invention, the medium chain aminocarboxylic acid is preferably 12-aminododecanoic acid having 12 carbon atoms, but the present invention is not limited thereto.

In the present invention, the substrate of step (2) may be a fatty acid, but the present invention is not limited thereto. According to an embodiment of the present invention, a fatty acid having 5 to 30 carbon atoms, preferably 8 to 16 carbon atoms, and more preferably dodecanoic acid having 12 carbon atoms may be used as the fatty acid, but the present invention is not limited thereto.

### [Mode for Invention]

Hereinafter, the present invention will be described in further detail with reference to examples thereof.

However, it should be understood that the following examples are just preferred examples for the purpose of illustration only and is not intended to limit or define the scope of the invention.

### Example 1: Construction of knock-out cassette

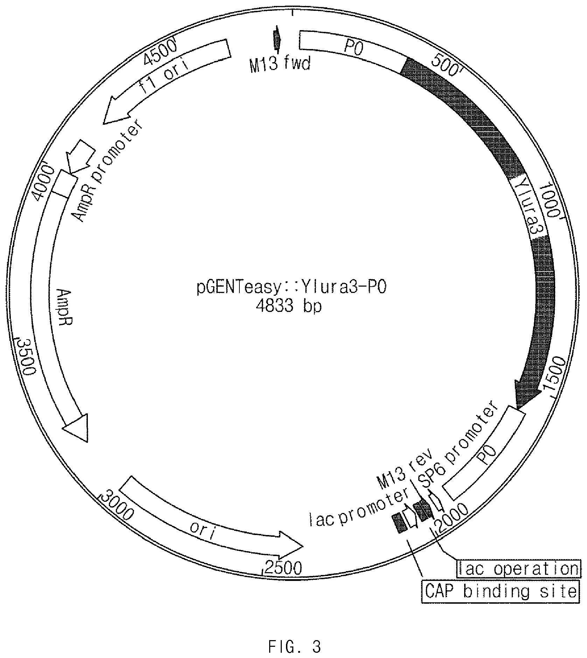

A vector containing an ura3 gene to be used as a selective marker for gene knockout to modify a strain, and a pop-out region for deleting the ura3 gene after insertion of a knock-out cassette was constructed (FIG. 3). A *Yarrowia-derived* gene was used as the ura3 gene, and the pop-out region used to modify a strain had a total of four sequences, and was referenced from two genes. Here, a *Bacillus-derived* glutamate-producing gene was used as one of the genes, and a gene associated with a *Salmonella-* or cloning vector pHUKH-derived His operon was used as the other one. The primers and sequences thereof used to construct the pop-out vectors are listed in the following Table 1.

**[Table 1]**

| Pop-out Vectors | | | |
|---|---|---|---|
| Names | | **Base Sequences** | **SEQ ID NOs** |
| **HisG1** | BglII F | aattgggcccagatctcagaccggttcagacaggat | 13 |
| | EcoRI R | tctctgggcggaattcggaggtgcggatatgaggta | 14 |
| | NotIF | tgTTTCTCGgcggccgccagaccggttcagacaggat | 15 |
| | BamHI R | TCCAACGCGTGGATCCggaggtgcggatatgaggta | 16 |
| **HisG2** | BglII F | aattgggcccagatctaacgctacctcgaccagaaa | 17 |
| | EcoRI R | tctctgggcggaattctcttctcgatcggcagtacc | 18 |
| | NotIF | tgTTTCTCGgcggccgcaacgctacctcgaccagaaa | 19 |
| | BamHI R | TCCAACGCG TGGATCCtcttctcgatcggcagtacc | 20 |
| **glt2** | BglII F | aattgggcccagatctTCAGAACTTGCGCCGATAAA | 21 |
| | EcoRI R | tctctgggcggaattcCTTTGCCAGCTAGACCATAGAG | 22 |
| | NotIF | tgTTTCTCGgcggccgcTCAGAACTTGCGCCGATAAA | 23 |
| | BamHI R | | 24 |
| **glt3** | BglII F | aattgggcccagatctATTGGCGGGTTCGTTACTT | 25 |
| | EcoRI R | tctctgggcggaattcCCTGGAAGAAGGCCGTATTATC | 26 |
| | NotIF | tgTTTCTCGgcggccgcATTGGCGGGTTCGTTACTT | 27 |
| | BamHI R | | 28 |

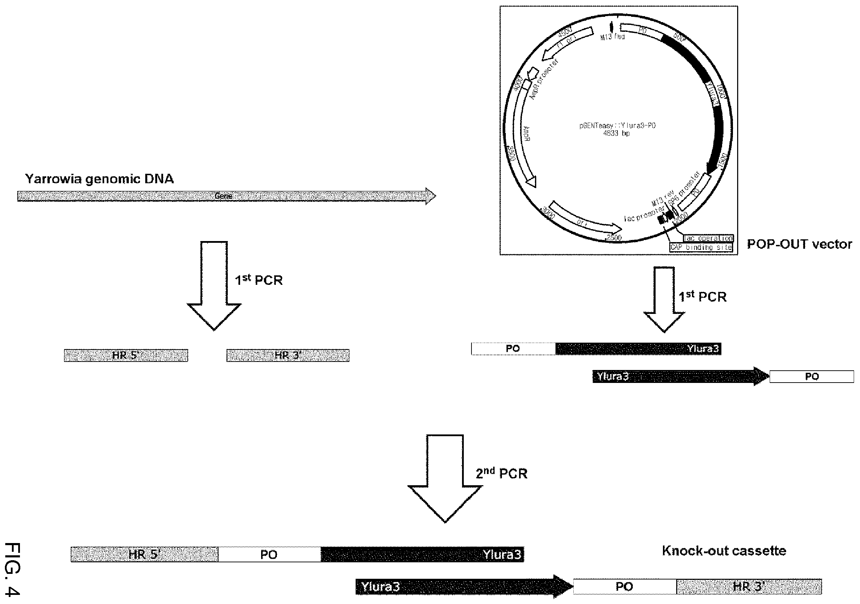

A knock-out cassette was constructed as shown in FIG. 4. First, PCR of a homologous region (HR) to be knocked out from the genomic DNA of *Yarrowia* sp., and PCR of two 5'- and 3'-terminal fragments from a pop-out vector were carried out separately. Thereafter, each of the 5' HR and 3' HR was subjected to alignment PCR (2^{nd} PCR) with a PO-ura3 region to construct a knock-out cassette. The primers and sequences thereof used to amplify the respective homologous regions are listed in Table 2.

**[Table 2]**

| Gene Deletions | | | |
|---|---|---|---|
| Names | | **Base Sequences** | **SEQ ID NOs** |
| **ACO1** | F1 | TTCCTCAATGGTGGAGAAGA | 29 |
| | R1 | | 30 |
| | F2 | | 31 |
| | R2 | AAGAAGGGCTTGAGAGTCG | 32 |
| **ACO2** | F1 | CCCAACAACACTGGCAC | 33 |
| | R1 | | 34 |
| | F2 | ATCGCTACCTCATATCCGCACCTCC gacaagacccgacaggc | 35 |
| | R2 | AGACCAGAGTCCTCTTCG | 36 |
| **ACO3** | F1 | Accttcacagagccaccca | 37 |
| | R1 | ATGGCTCTCTGGGCGgtgttgggggtgttgatgatg | 38 |
| | F2 | TTGTTGTGTTTCTCGcaaggttctcatcgaggcctg | 39 |
| | R2 | Aggaaaggtcgaagagtgctct | 40 |
| **ACO4** | F1 | Actgcgagagcgatctg | 41 |
| | R1 | | 42 |
| | F2 | ATCGCTACCTCATATCCGCACCTCC gaggacgacaaagccggag | 43 |
| | R2 | AGAGCAGAGTCCTCCTCAA | 44 |
| **ACO5** | F1 | AACTTCCTCACAGGCAGCGAGC | 45 |
| | R1 | | 46 |
| | F2 | ttgttgtgtttctcg ccccgtcaaggacgctgag | 47 |
| | R2 | ACAGTAAGGTGGGGCTTGACTC | 48 |
| **ACO6** | F1 | AGTCCCTCAACACGTTTACCG | 49 |
| | R1 | | 50 |
| | F2 | | 51 |
| | R2 | AGGAAGGGTCTAATGACAGA | 52 |
| **FALD H1** | F1 | AATCACTCCTCCTACGC | 53 |
| | R1 | | 54 |
| | F2 | | 55 |
| | R2 | ACCGACATAATCTGAGCAAT | 56 |
| **FALD H2** | F1 | Accactaggtgagatcgag | 57 |
| | R1 | | 58 |
| | F2 | | 59 |
| | R2 | GATCACCCAGAACCATAGC | 60 |
| **FALD H3** | F1 | GTGACCCCCACCACGTCAC | 61 |
| | R1 | | 62 |
| | F2 | | 63 |
| | R2 | CAGGGCTGGGGACCACC | 64 |
| **FALD H4** | F1 | TACCGACTGGACCAGATTC | 65 |
| | R1 | | 66 |
| | F2 | | 67 |
| | R2 | CAAATCTTTCGGAAGATTCGG | 68 |

The primers used to PCR-amplify the pop-out region and ura3 as two fragments are listed in Table 3.

**[Table 3]**

| Pop-out Cassettes | | | |
|---|---|---|---|
| Names | | **Base Sequences** | **SEQ ID NOs** |
| **HISG1** | F | cagaccggttcagacaggat | 69 |
| | R | ggaggtgcggatatgaggta | 70 |
| **HISG2** | F | aacgctacctcgaccagaaa | 71 |
| | R | tcttctcgatcggcagtacc | 72 |
| **glt2** | F | TCAGAACTTGCGCCGATAAA | 73 |
| | R | CTTTGCCAGCTAGACCATAGAG | 74 |
| **glt3** | F | ATTGGCGGGTTCGTTACTT | 75 |
| | R | CCTGGAAGAAGGCCGTATTATC | 76 |
| **Bipartite** | Ulura3 cs 2B | Atgccctcctacgaagctcgagc | 77 |
| | Ylura3F | Ctcccaacgagaagctggcc | 78 |

### Example 2: Construction of transduction vector

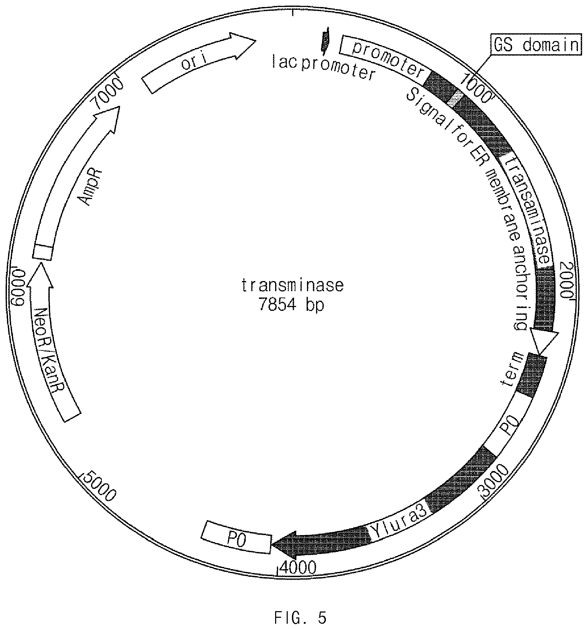

To insert an ω-transaminase into a *Yarrowia* strain, a vector as shown in FIG. 5 was constructed. The primers used for this purpose are listed in Table 4.

**[Table 4]**

| Transaminase Vectors | | |
|---|---|---|
| Names | **Base Sequences** | SEQ ID NOs |
| **EXP1-F** | ccaagcttggtaccgagctcaGagtttggcgcccgttttttc | 79 |
| **EXP1-R** | | 80 |
| **TEF-F** | ccaagcttggtaccgagctcaaactttggcaaagaggctgca | 81 |
| **TEF-R** | | 82 |
| **ALK1-F** | ccaagcttggtaccgagctcagatctgtgcgcctctacagaccc | 83 |
| **ALK1-R** | CGTTGTTTTTGCATATGagtgcaggagtattctggggagga | 84 |
| **XPR2t-F2** | gtcgacgcaattaacagatagtttgccg | 85 |
| **XPR2t- R3** | ctcgagggatcccggaaaacaaaacacgacag | 86 |
| **TA-F** | CATATGCAAAAACAACGTACTACCTCCC | 87 |
| **TA-R** | gtcgacTTAGGCCAAACCACGGGCTTTC | 88 |
| **ATATG2-ER-F** | actcctgcactCATatgtccaacgccctcaacctg | 89 |
| **XTATG2-ER-F** | ccaatccaacacatatgtccaacgccctcaacctg | 90 |
| **ER-R-1** | | 91 |
| **ER-R-2** | | 92 |
| **ETATG2-ER-1** | tgattacgccaagcttGagtttggcgcccgttttttc | 93 |
| **ETATG2-ER-2** | | 94 |
| **TTATG2-ER-1** | taattacaccaaacttaaactttggcaaagaggctg | 95 |
| **TTATG2-ER-2** | acaggttgagggcgttggacatATGtttgaatgattcttatactcagaag | 96 |
| **ER-F** | atgtccaacgccctcaacctg | 97 |
| **ER-R-3** | CGTTGTTTTTGCATAGAACCGCCACCGCCGCTAC | 98 |

The transaminase cassettes were constructed in the same manner as in FIG. 4, except that, when two fragments of PCR products were obtained from the vector, the genes spanning from a promoter to ura3 were amplified to construct the cassettes. The primers used to construct the cassettes are listed in the following Table 5.

**[Table 5]**

| **Transaminase Cassettes** | | |
|---|---|---|
| **Names** | **Base Sequences** | **SEQ ID NOs** |
| **TA-FALDH4-F1** | TACCGACTGGACCAGATTC | 99 |
| **TA-FALDH4-R1** | CGGCAGTGGCAATGATCTTAC | 100 |
| **TA-FALDH4-F2** | ctcctctatggtctagctggcaaagACTCGATTCATCGCTCCTAC | 101 |
| **TA-FALDH4-R2** | CAAATCTTTCGGAAGATTCGG | 102 |
| **ATATG2-F** | gtcggtaagatcattgccactgccgagatctgtgcgcctctacagac | 103 |
| **ETATG2-F** | gtcggtaagatcattgccactgccgGagtttggcgcccgttttttc | 104 |
| **TTATG2-F** | gtcggtaagatcattgccactgccgaaactttggcaaagaggctgc | 105 |
| **XTATG2-F** | gtcggtaagatcattgccactgccgacgcgtggagagtttgggtt | 106 |

The gene sequences used to modify the recombinant microorganism strain according to the present invention are listed in the sequence listing, and summarized in Table 6.

**[Table 6]**

| **Genes** | **SEQ ID NOs** | **Genes** | **SEQ ID NOs** |
|---|---|---|---|
| FALDH1 | 1 | ACO3 | 7 |
| FALDH2 | 2 | ACO4 | 8 |
| FALDH3 | 3 | ACO5 | 9 |
| FALDH4 | 4 | ACO6 | 10 |
| ACO1 | 5 | ω-transaminase | 11 |
| ACO2 | 6 | Ura3 | 12 |

### Example 3: Preparation of recombinant microorganism strain

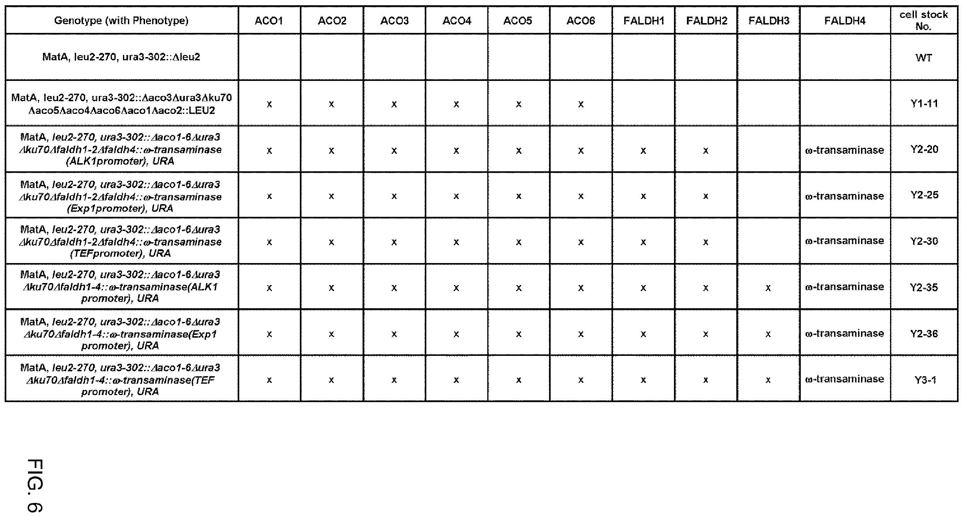

The knock-out cassette constructed in Example 1 and the transduction vector constructed in Example 2 were used to prepare a total of eight knock-out strains from which some of all of a fatty aldehyde dehydrogenase gene in an ω-oxidative metabolism pathway present in a wild-type *Yarrowia* strain and β-oxidative metabolism pathway-related genes were deleted and into which an ω-transaminase gene was also introduced (FIG. 6). Specifically, a strain in which a gene was to be knocked out or be introduced was plated on an YPD plate, and cultured at 30 °C for 16 to 24 hours. The cultured cells were scraped with a loop, put into 100 µL of a one-step buffer (45% PEG4000, 100 mM DTT, 0.1 L of LiAc, 25 µg of single-strand carrier DNA), and vortexed. Thereafter, the knock-out cassette and the transduction vector (1 ng or more) were added thereto, and the resulting mixture was vortexed again, and then cultured at 39 °C for an hour. The cultured sample was loaded onto a selective medium (6.7 g/L of YNB without amino acids, and 20 g/L of glucose), and then cultured at 30 °C for 48 hours to screen the strains into which the constructed cassette was inserted. To check whether the cassettes were correctly inserted onto the genomes of the screened strains, PCR was then performed using the primers included in the gene deletions listed in Table 2.

To insert another cassette, a pop-out process was performed on the strain into which the cassette was inserted. The strain screened from the selective medium was inoculated in 2 mL of an YPD medium, and cultured at 30 °C for 16 hours, and 200 µL of the culture broth was then spread on a 5' FOA medium (6.7 g/L of YNB without amino acids, 20 g/L of glucose, 0.8 g/L of 5' FOA, 0.1 g/L of uracil, and 0.1 g/L of uridine), and then cultured at 30 °C for 48 hours. The strains grown on the 5' FOA medium were picked, and spread on an YPD plate and a UD plate to screen the strains grown on the YPD plate. Also, a PCR process was again performed using the primers listed in Table 2 to check whether the ura3 gene was deleted from the strains. A knock-out process was performed on other genes of the Ura3-free strains.

### Example 4: Culturing of recombinant microorganism strain

A day earlier, the strain to be cultured and tested was inoculated in 2 mL of an YPD medium (Bacto Laboratories, 10 g/L of Yeast extract, 20 g/L of peptone, and 20 g/L of glucose), and grown at 30 °C and 200 rpm for a day. 2 mL of a growth medium (pH 6.0) having the compositions listed in Table 7 was put into a 24-well plate, and a pre-cultured culture broth was inoculated at 1%. Thereafter, the strains were cultured at 30 °C and 450 rpm for a day in a plate stirrer. The strains cultured for a day were inoculated at a volume of 900 µL in a new plate containing 900 µL of a conversion medium (pH 7.6) listed in Table 8, and 200 µL of a substrate was added thereto at the same time. The resulting mixture was cultured at 30 °C and 450 rpm for a day. In this case, 10 g/L of dodecanoic acid dissolved in DMSO was used as the substrate.

**[Table 7]**

| **Growth Medium (pH 6.0)** | | |
|---|---|---|
| Components | | Concentration (g/L) |
| Glucose | | 50 |
| YNB w/o amino acid | | 6.7 |
| Yeast extract | | 10 |
| (NH₄)₂SO₄ | | 5 |
| Uracil | | 0.05 |
| 0.1 M phosphate buffer | | |

| Preparation of 0.1 M potassium phosphate buffer at 25 °C | | |
|---|---|---|
| pH | Volume (mL) of 1 M K₂HPO₄ | Volume (mL) of 1 M KH₂PO₄ |
| 6.0 | 13.2 | 86.8 |

**[Table 8]**

| **Conversion Medium (pH 7.6)** | | |
|---|---|---|
| Components | | Concentration (g/L) |
| Glucose | | 30 |
| YNB w/o amino acid | | 6.7 |
| Yeast extract | | 3 |
| (NH₄)₂SO₄ | | 15 |
| Uracil | | 0.05 |
| L-alanine | | 10 |
| 0.1 M phosphate buffer | | |

| Preparation of 0.1 M potassium phosphate buffer at 25 °C | | |
|---|---|---|
| pH | Volume (mL) of 1 M K₂HPO₄ | Volume (mL) of 1 M KH₂PO₄ |
| 7.6 | 86.6 | 13.4 |

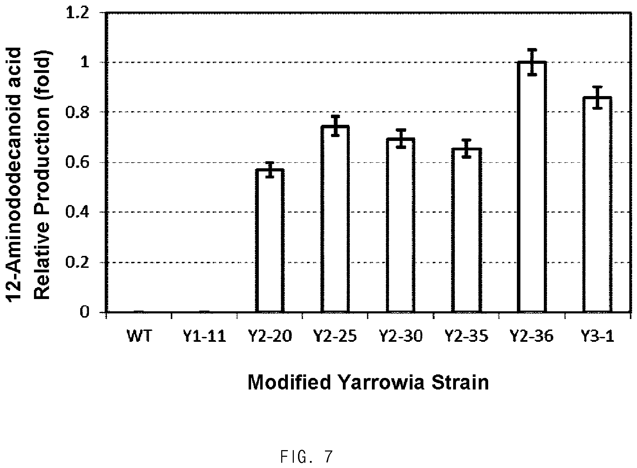

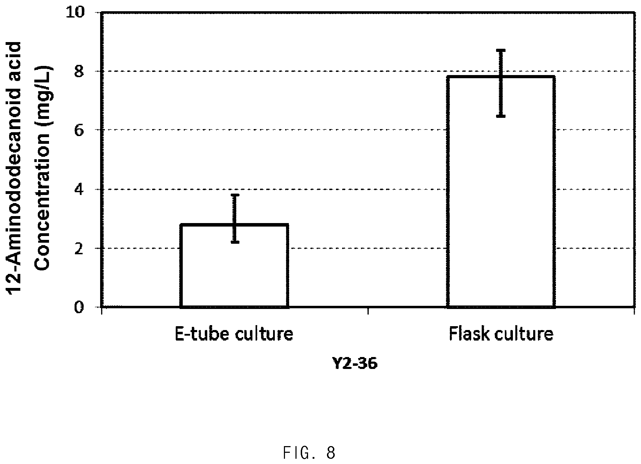

As a result, it was revealed that the Y1-11 strain in which only the β-oxidative metabolism pathway-related genes were knocked out did not produce 12-aminododecanoic acid from dodecanoic acid serving as the substrate, but all the Y2-20, Y-2-25, Y2-30, Y2-35, Y2-36 and Y3-1 strains in which the fatty aldehyde dehydrogenase gene were further knocked out and into which the ω-transaminase was introduced exhibited an excellent ability to synthesize 12-aminododecanoic acid (FIG. 7). Also, it was revealed that the Y2-36 strain exhibited an ability to synthesize approximately 8 mg/L of 12-aminododecanoic acid when cultured in the flask (FIG. 8). In the following experiment, a sample analysis test was performed using the Y2-36 strain.

### Example 5: Sample Analysis

100 µL of 6 N sulfuric acid was added to 500 µL of a culture broth of the Y2-36 strain, which had been proven to have the most excellent ability to synthesize 12-aminododecanoic acid in Example 4, and 500 µL of methanol containing 10% toluene and 2.2% hydrochloric acid was added thereto to perform a methylation reaction. Thereafter, a methylation reaction was performed at 100 °C for an hour. 100 µL of 10 N sodium hydroxide and 500 µL of diethyl ether were added to the reaction solution, and the resulting mixture was thoroughly vortexed, and then centrifuged at 12,000 rpm for 2 minutes. Then, a GC/MS assay was performed under the following analytical conditions to separate only a solvent layer.

### Analytical Conditions

① Equipment: Agilent 5975 MSD
② Column: HP-5MS
③ Temperature: Oven(150 °C to 230 °C)
④ Carrier Gas: He
⑤ Flow Rate: 1 mL/min.

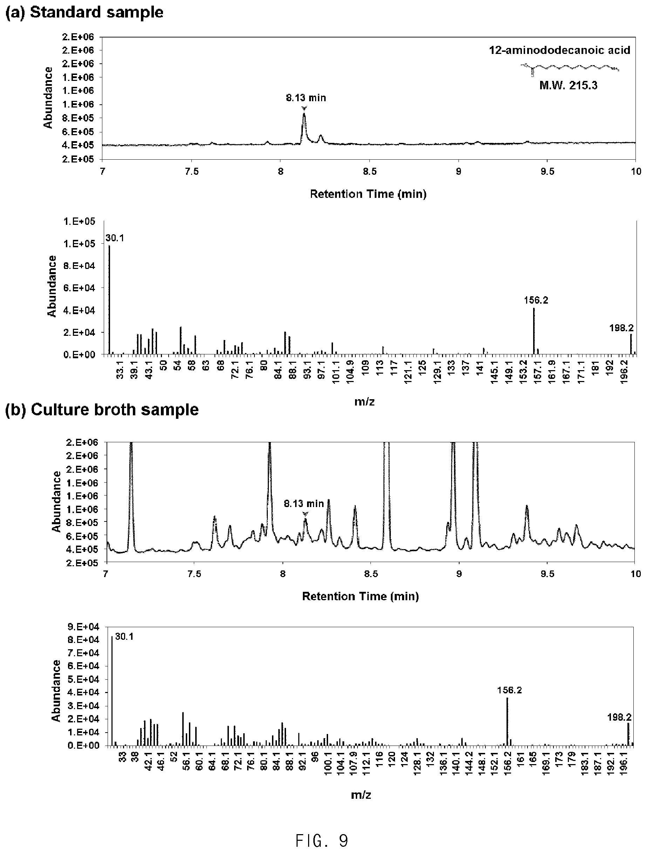

As a result, it was confirmed that the recombinant Y2-36 strain of the present invention was able to synthesize 12-aminododecanoic acid from dodecanoic acid serving as the substrate (FIG. 9).
## Claims

1. A recombinant microorganism from which a fatty aldehyde dehydrogenase gene in an ω-oxidative metabolism pathway and β-oxidative metabolism pathway-related genes are deleted and into which an ω-transaminase gene is also introduced.

2. The recombinant microorganism of claim 1, wherein the fatty aldehyde dehydrogenase gene and the β-oxidative metabolism pathway-related genes are deleted from all homologous genes present in the microorganism.

3. The recombinant microorganism of claim 1, wherein the fatty aldehyde dehydrogenase gene and the β-oxidative metabolism pathway-related genes are deleted from some of the homologous genes present in the corresponding microorganism.

4. The recombinant microorganism of claim 1, wherein the fatty aldehyde dehydrogenase gene is selected from the group consisting of FALDH1, FALDH2, FALDH3, and FALDH4 genes, and preferably each of the FALDH1, FALDH2, FALDH3, and FALDH4 genes comprise base sequences set forth in SEQ ID NOs: 1 to 4.

5. The recombinant microorganism of claim 1, wherein the β-oxidative metabolism pathway-related gene is an acyl-CoA oxidase gene.

6. The recombinant microorganism of claim 5, wherein the acyl-CoA oxidase gene is selected from the group consisting of ACO1, ACO2, ACO3, ACO4, ACO5, and ACO6 genes, and preferably each of the ACO1, ACO2, ACO3, ACO4, ACO5, and ACO6 genes comprise base sequences set forth in SEQ ID NOs: 5 to 10, respectively.

7. The recombinant microorganism of claim 1, wherein the ω-transaminase gene comprises a base sequence set forth in SEQ ID NO: 11.

8. The recombinant microorganism of claim 1, wherein the microorganism is a yeast or *Escherichia coli.*

9. The recombinant microorganism of claim 8, wherein the yeast is selected from the group of the yeast consisting of *Yarrowia* sp., *Saccharomyces* sp., *Pichia* sp., and *Candida* sp.

10. The recombinant microorganism of claim 9, wherein the yeast of *Yarrowia* sp. is *Yarrowia lipolytica.*

11. A method for producing a medium chain aminocarboxylic acid, comprising:
(1) preparing the recombinant microorganism according to any one of claims 1 to 10; and
(2) treating the recombinant microorganism with a substrate to culture the recombinant microorganism.

12. The method of claim 11, wherein the substrate is a fatty acid, and preferably the fatty acid is a fatty acid having 5 to 30 carbon atoms.

13. The method of claim 12, wherein the fatty acid is dodecanoic acid.

14. The method of claim 11, wherein the medium chain aminocarboxylic acid is a medium chain aminocarboxylic acid compound having 5 to 30 carbon atoms.

15. The method of claim 14, wherein the medium chain aminocarboxylic acid is 12-aminododecanoic acid.

## Patentansprüche

1. Ein rekombinanter Mikroorganismus, aus dem ein Fettaldehyd-Dehydrogenase-Gen in einem ω-oxidativen Stoffwechselweg und zum β-oxidativen Stoffwechselweg gehörenden Gene entfernt werden und in den auch ein ω-Transaminase-Gen eingeführt wird.

2. Rekombinanter Mikroorganismus nach Anspruch 1, wobei das Fettaldehyd-Dehydrogenase-Gen und die zum β-oxidativen Stoffwechselweg gehörenden Gene aus allen homologen Genen entfernt werden, die im Mikroorganismus vorhanden sind.

3. Rekombinanter Mikroorganismus nach Anspruch 1, wobei das Fettaldehyd-Dehydrogenase-Gen und die zum β-oxidativen Stoffwechselweg gehörenden Gene aus einigen homologen Genen entfernt werden, die im entsprechenden Mikroorganismus vorhanden sind.

4. Rekombinanter Mikroorganismus nach Anspruch 1, wobei das Fettaldehyd-Dehydrogenase-Gen aus der Gruppe ausgewählt wird, die aus FALDH1, FALDH2, FALDH3 und FALDH4-Genen besteht und wobei vorzugsweise jedes der FALDH1, FALDH2, FALDH3 und FALDH4-Gene Basissequenzen enthält, die in SEQ ID NO: 1 bis 4 wiedergegeben sind.

5. Rekombinanter Mikroorganismus nach Anspruch 1, wobei das zum β-oxidativen Stoffwechselweg gehörenden Gen ein Acyl-CoA-Oxidase-Gen ist.

6. Rekombinanter Mikroorganismus nach Anspruch 5, wobei das Acyl-CoA-Oxidase-Gen aus der Gruppe ausgewählt wird, die aus ACO1, ACO2, ACO3, ACO4, ACO5 und ACO6-Genen besteht und wobei vorzugsweise jedes der ACO1, ACO2, ACO3, ACO4, ACO5 und ACO6-Gene Basissequenzen enthält, die jeweils in SEQ ID NO: 5 bis 10 wiedergegeben sind.

7. Rekombinanter Mikroorganismus nach Anspruch 1, wobei das ω-transaminase-Gen eine Basissequenz enthält, die in SEQ ID NO: 11 wiedergegeben ist.

8. Rekombinanter Mikroorganismus nach Anspruch 1, wobei der Mikroorganismus eine Hefe oder *Escherichia coli* ist.

9. Rekombinanter Mikroorganismus nach Anspruch 8, wobei die Hefe aus der Hefe-Gruppe ausgewählt wird, die aus *Yarrowia* sp., *Saccharomyces* sp., *Pichia* sp., und *Candida* sp. besteht.

10. Rekombinanter Mikroorganismus nach Anspruch 9, wobei die *Yarrowia* sp.-Hefe *Yarrowia lipolytica* ist.

11. Ein Verfahren zur Herstellung von mittelkettiger Aminocarbonsäure, bestehend aus:
(1) Herstellung des rekombinanten Mikroorganismus nach einem der Ansprüche 1 bis 10; und
(2) Behandlung des rekombinanten Mikroorganismus mit einem Substrat zur Kultivierung des rekombinanten Mikroorganismus.

12. Verfahren nach Anspruch 11, wobei das Substrat eine Fettsäure ist, wobei die Fettsäure vorzugsweise eine Fettsäure mit 5 bis 30 Kohlenstoffatomen ist.

13. Verfahren nach Anspruch 12, wobei die Fettsäure eine Dodekansäure ist.

14. Verfahren nach Anspruch 11, wobei die mittelkettige Aminocarbonsäure eine Aminocarbonsäureverbindung mit 5 bis 30 Kohlenstoffatomen ist.

15. Verfahren nach Anspruch 14, wobei die mittelkettige Aminocarbonsäure eine 12-Aminododekansäure ist.

## Revendications

1. Micro-organisme recombinant dans lequel un gène de «fatty» aldéhyde déshydrogénase d'une voie métabolique ω-oxydative et des gènes relatifs à la voie métabolique β-oxydative sont délétés et dans lequel un gène de ω-transaminase est aussi introduit.

2. Micro-organisme recombinant selon la revendication 1, dans lequel le gène de la « fatty » aldéhyde déshydrogénase et les gènes relatifs à la voie métabolique β-oxydative sont délétés de tous les gènes homologues présents dans le micro-organisme.

3. Micro-organisme recombinant selon la revendication 1, dans lequel le gène de la « fatty » aldéhyde déshydrogénase et les gènes relatifs à la voie métabolique β-oxydative sont délétés dans une partie des gènes homologues présents dans le micro-organisme correspondant.

4. Micro-organisme recombinant selon la revendication 1, dans lequel le gène de la « fatty » aldéhyde déshydrogénase est sélectionné parmi le groupe constitué des gènes FALDH1, FALDH2, FALDH3, et FALDH4, et de préférence chacun des gènes FALDH1, FALDH2, FALDH3, et FALDH4 comprenant des séquences en bases décrites dans SEQ ID NOs : 1 à 4, respectivement.

5. Micro-organisme recombinant selon la revendication 1, dans lequel le gène relatif à la voie métabolique β-oxydative est un gène d'acyl-CoA oxydase.

6. Micro-organisme recombinant selon la revendication 5, dans lequel le gène d'acyl-CoA oxydase est sélectionné parmi le groupe constitué des gènes ACO1, ACO2, ACO3, ACO4, ACO5, et ACO6, et de préférence chacun des gènes ACO1, ACO2, ACO3, ACO4, ACO5, et ACO6 comprenant des séquences en bases décrites dans SEQ ID NOs : 5 à 10, respectivement.

7. Micro-organisme recombinant selon la revendication 1, dans lequel le gène de ω-transaminase comprend une séquence en bases décrite dans SEQ ID NO:11.

8. Micro-organisme recombinant selon la revendication 1, dans lequel le micro-organisme est une levure ou *Escherichia coli.*

9. Micro-organisme recombinant selon la revendication 8, dans lequel la levure est sélectionnée parmi le groupe de levures constitué de *Yarrowia* sp., *Saccharomyces* sp., *Pichia* sp., et *Candida* sp.

10. Micro-organisme recombinant selon la revendication 9, dans lequel la levure de *Yarrowia* sp. est *Yarrowia lipolytica.*

11. Méthode de production d'un acide aminocarboxylique à chaîne moyenne comprenant :
(1) préparation d'un micro-organisme recombinant selon l'une des revendications 1 à 10 ; et
(2) traitement du micro-organisme recombinant avec un substrat pour cultiver le micro-organisme recombinant.

12. Méthode selon la revendication 11, dans laquelle le substrat est un acide gras, et de préférence l'acide gras est un acide gras ayant de 5 à 30 atomes de carbone.

13. Méthode selon la revendication 12, dans laquelle l'acide gras est de l'acide dodécanoïque.

14. Méthode selon la revendication 11, dans laquelle l'acide aminocarboxylique à chaîne moyenne est un composé d'acide aminocarboxylique à chaîne moyenne ayant de 5 à 30 atomes de carbone.

15. Méthode selon la revendication 14, dans laquelle l'acide aminocarboxylique à chaîne moyenne est l'acide 12-aminododécanoïque.
